# EUROPEAN PATENT APPLICATION

(11) **EP 3 981 353 A1**
(43) Date of publication of application: **13.04.2022**
(21) Application number: 21198469.5
(22) Date of filing: 23.09.2021
(51) Int. Cl.: A61B 34/30, A61B 34/00, A61B 46/00, A61B 90/00, A61B 17/00, A61B 18/00

(54) **SURGICAL INSTRUMENT**

(30) Priority: 07.10.2020 JP 2020169985
(71) Applicant: Medicaroid Corporation, Kobe-shi, Hyogo 650-0047 (JP)
(72) Inventor: TAKAHASHI, Kaoru, Hyogo, 650-0047 (JP); USUKI, Yu, Hyogo, 650-0047 (JP)
(74) Representative: Müller Hoffmann & Partner

(57) **Abstract**

A surgical instrument includes a conductive distal support member, a conductive proximal support member, a sealing mechanism being in contact with the proximal support member and including at least a part thereof having conductivity, and an electric wire. The electric wire includes an end effector-side end portion thereof being connected to the sealing mechanism to supply electrical energy to the end effector through the sealing mechanism, the proximal support body, and the distal support body.

## Description

### TECHNICAL FIELD

The invention may relate to a surgical instrument and may especially relate to an electrosurgical instrument.

### BACKGROUND ART

In a related art, there is known an electrosurgical instrument that includes a conductive distal clevis, a conductive proximal clevis, and a conductive end effector.

U.S. Patent No. 7,824,401 discloses a surgical instrument including a shaft, a seal, and an electric wire.

In the surgical instrument disclosed in U.S. Patent No. 7,824,401, an end effector is rotatably attached to a distal clevis. The distal clevis is rotatably attached to a proximal clevis. The proximal clevis is connected to a distal end portion (a tip portion) of the shaft. The seal is provided inside of the shaft. Specifically, the seal is provided between the distal end portion of the shaft (an end effector-side end portion of the shaft) and the proximal clevis. The electric wire is provided for supplying electrical energy to the end effector therethrough. An end effector-side end of the electric wire is connected to the proximal clevis.

In the surgical instrument disclosed in U.S. Patent No. 7,824,401, the electricity flowing through the electric wire is supplied to the end effector via the proximal clevis and the distal clevis.

### SUMMARY

However, in the surgical instrument disclosed in U.S. Patent No. 7,824,401, the electric wire is terminated at the proximal clevis, and thus the electric wire passes through the seal provided between the distal end portion of the shaft and the proximal clevis. Therefore, in the surgical instrument disclosed in U.S. Patent No. 7,824,401, the seal is formed with a though hole through which the electric wire passes and the sealing performance of the seal may be deteriorated.

An object of the invention may be to provide a surgical instrument that is capable of enhancing a sealing performance of a sealing mechanism thereof.

An aspect of the invention may be a surgical instrument to be attached to a robot arm. The surgical instrument may include a distal support body, a proximal support body, a shaft, a sealing mechanism, and an electric wire. The distal support body has conductivity and supports the end effector to be rotatable about a first axis with respect to the distal support body. The proximal support body has conductivity and supports the distal support body to be rotatable about a second axis with respect to the proximal support body. The shaft is formed in a tubular shape and is to be connected to the proximal support body. The sealing mechanism is in contact with the proximal support body and includes at least a part thereof having conductivity. The electric wire includes an end effector-side end portion thereof being connected to the sealing mechanism to supply electrical energy to the end effector through the sealing mechanism, the proximal support body and the distal support body.

As described above, according to the aspect of the invention, the sealing mechanism is in contact with the proximal support body and includes at least a part thereof having conductivity, and the electric wire includes the end effector-side end connected to the sealing mechanism. Accordingly, the electricity can be supplied from the electric wire to the proximal support body via the sealing mechanism, without connecting the end effector-side end portion of the electric wire with the proximal support body. Therefore, the electric wire does not need to pass through the sealing mechanism. That is, the sealing mechanism does not need to have a through hole through which the electric wire passes. As a result, the sealing performance of the sealing mechanism can be improved.

According to the invention, the sealing performance of the sealing mechanism of the surgical instrument can be improved.

### BRIEF DESCRIPTION OF THE DRAWINGS

FIG. 1 is a diagram illustrating an overview of a robotic surgical system according to first to fourth embodiments;
FIG. 2 is a block diagram illustrating a view of a control-related configuration of the robotic surgical system according to first to fourth embodiments;
FIG. 3 is a diagram illustrating a perspective view of a state where a surgical instrument is attached to a robot arm via an adaptor according to first to fourth embodiments;
FIG. 4 is a diagram illustrating an exploded perspective view of a state where the surgical instrument is to be attached to the robot arm via the adaptor according to first to fourth embodiments;
FIG. 5 is a diagram illustrating a perspective view of the surgical instrument according to first to fourth embodiments;
FIG. 6 is a diagram illustrating a perspective view of the adaptor according to first to fourth embodiments;
FIG. 7 is a diagram illustrating a perspective view of a shaft and an end effector according to first to fourth embodiments;
FIG. 8 is a diagram illustrating an exploded perspective view of the surgical instrument according to a first embodiment as seen from a Y2 direction;
FIG. 9 is a diagram illustrating a perspective view of the shaft according to a first embodiment;
FIG. 10 is a diagram illustrating an exploded perspective view of the surgical instrument according to a first embodiment as seen from a Y1 direction;
FIG. 11 is a diagram illustrating a perspective view of a retainer according to a first embodiment;
FIG. 12 is a diagram illustrating a cross sectional view of a connection portion between a proximal clevis and the shaft of the surgical instrument according to a first embodiment;
FIG. 13 is a diagram illustrating a perspective view of a seal member according to a first embodiment;
FIG. 14 is a diagram of a schematic view illustrating an electrical connection between the proximal clevis and the retainer and an electrical connection between the proximal clevis and a distal clevis of the surgical instrument according to a first embodiment;
FIG. 15 is a diagram illustrating a cross sectional view of a connection structure from the shaft to the end effector of the surgical instrument according to a first embodiment;
FIG. 16 is a diagram illustrating a perspective view of an electrical connection between the end effector and the distal clevis of the surgical instrument according to a first embodiment;
FIG. 17 is a diagram of a cross sectional view illustrating a connection portion between a proximal clevis and a shaft of a surgical instrument according to a second embodiment;
FIG. 18 is a diagram of a cross sectional view illustrating a connection portion between a proximal clevis and a shaft of a surgical instrument according to a third embodiment; and
FIG. 19 is a diagram of a cross sectional view illustrating a connection portion between a proximal clevis and a shaft of a surgical instrument according to a fourth embodiment.

### DETAILED DESCRIPTION

Descriptions are provided hereinbelow for one or more embodiments based on the drawings. In the respective drawings referenced herein, the same constituents are designated by the same reference numerals and duplicate explanation concerning the same constituents is omitted. All of the drawings are provided to illustrate the respective examples only.

### [First Embodiment]

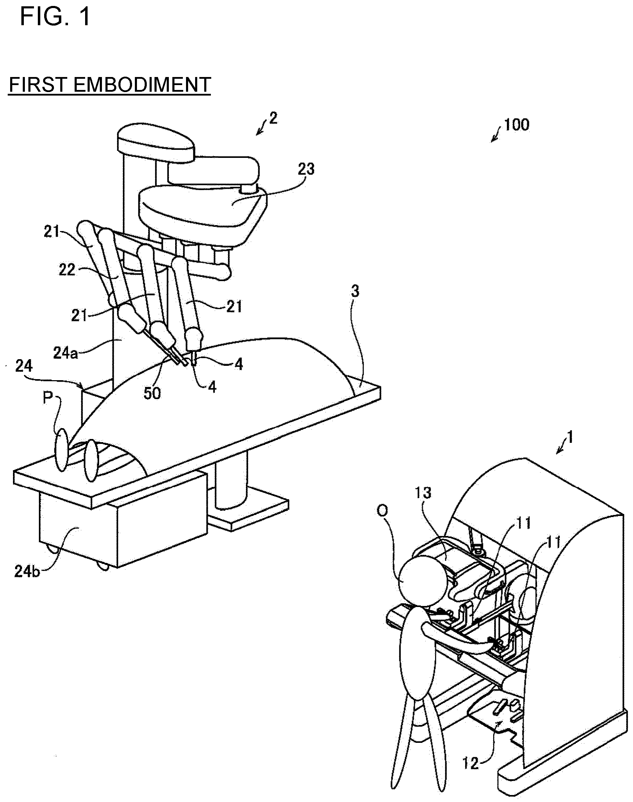

With reference to FIGS. 1 to 16, a configuration of a surgical instrument according to a first embodiment of the invention is described.

### (Configuration of Robotic Surgical System)

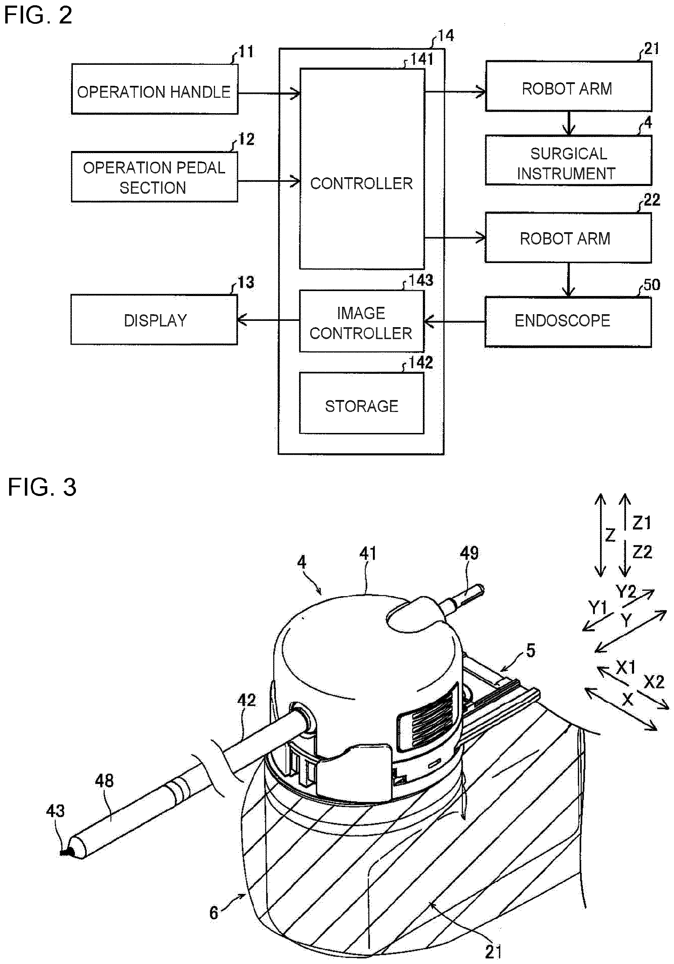

A configuration of a robotic surgical system 100 is described with reference to FIGS. 1 and 2.

As illustrated in FIG. 1, the robotic surgical system 100 includes a remote control apparatus 1 and a patient-side apparatus 2. The remote control apparatus 1 is provided to remotely control medical equipment provided to the patient-side apparatus 2. When an operator O, as a surgeon, inputs an action mode instruction to be executed by the patient-side apparatus 2, to the remote control apparatus 1, the remote control apparatus 1 transmits the action mode instruction to the patient-side apparatus 2. In response to the action mode instruction transmitted from the remote control apparatus 1, the patient-side apparatus 2 operates the medical equipment, including surgical instruments 4 attached to robot arms 21 and an endoscope 50 attached to a robot arm 22. This allows minimally invasive surgery.

The patient-side apparatus 2 is positioned beside an operation table 3 on which a patient P is to be laid. The patient-side apparatus 2 constitutes an interface to perform surgery for the patient P in response to an input from the remote control apparatus 1. The patient-side apparatus 2 includes plural robot arms 21, a robot arm 22, a platform 23, a positioner 24, and a controller (not illustrated).

Each of the plural robot arms 21 includes plural joints. Each joint of the robot arm 21 includes a driver (not illustrated) provided with a servo-motor and a position detector such as an encoder. The robot arms 21 are configured so that the medical equipment attached to each robot arm 21 is controlled by a driving signal given through the controller and performs a desired movement. Note that the robot arm 22 has a configuration same as the robot arm 21.

The surgical instruments 4 as the medical equipment are detachably attached to distal end portions of the robot arms 21. In surgery using the patient-side apparatus 2, the robot arms 21 introduce the surgical instruments 4 into a body of the patient P through a cannula (trocar) placed on a body surface of the patient P.

Each surgical instrument 4 includes: a housing 41 (see FIG. 3), which is attached to the robot arm 21; an elongated shaft 42 (see FIG. 3); and an end effector 43 (see FIG. 3), which is provided at a tip portion (a distal end portion) of the shaft 42. The end effector 43 of the surgical instrument 4 is located near the surgery site (treatment site).

To the distal end of the robot arm 22, the endoscope 50 as medical equipment is detachably attached. The endoscope 50 captures an image in a body cavity of the patient P. The captured image is outputted to the remote control apparatus 1. The endoscope 50 is a 3D endoscope capable of capturing a three-dimensional image or a 2D endoscope. In surgery using the patient-side apparatus 2, the robot arm 22 introduces the endoscope 50 into the body of the patient P through the trocar placed on the body surface of the patient P. The endoscope 50 is then located near the surgery site.

The platform 23 commonly supports the robot arms 21 and the robot arm 22. The positioner 24 is placed on the floor of an operation room and supports the platform 23. The positioner 24 includes a column 24a including an elevating shaft adjustable in the vertical direction and a base 24b connected the column 24a and including wheels and thus being movable on the floor surface.

The remote control apparatus 1 constitutes the interface with the operator O. The remote control apparatus 1 is an apparatus that allows the operator O to operate the surgical instruments 4 attached to the robot arms 21 and the endoscope 50 attached to the robot arm 22. Specifically, the remote control apparatus 1 is configured to transmit action mode instructions which are inputted by the operator O and are to be executed by the surgical instruments 4 and endoscope 50, to the patient-side apparatus 2 through the controller. The remote control apparatus 1 is installed beside the operation table 3 so that the operator O can see the condition of the patient P very well while operating the remote control apparatus 1, for example. The remote control apparatus 1 may be configured to transmit action mode instructions wirelessly and installed in a room different from the operation room where the operation table 3 is installed.

The action modes to be executed by the surgical instruments 4 include modes of actions to be taken by each surgical instrument 4 (a series of positions and postures) and actions to be executed by the function of each surgical instrument 4. When the surgical instrument 4 is a pair of grasping forceps, for example, the action modes to be executed by the surgical instrument 4 include roll and pitch positions of the wrist of the end effector 43 and actions to open and close the jaws. When the surgical instrument 4 is a high-frequency knife, the action modes to be executed by the surgical instrument 4 may include vibration of the high-frequency knife, specifically, supply of current to the high-frequency knife. When the surgical instrument 4 is a snare wire, the action modes to be executed by the surgical instrument 4 may include a capturing action and an action to release the captured object. Further, the action modes may include an action to supply current to a bipolar or monopolar instrument to burn off the surgery site.

The action modes to be executed by the endoscope 50 include, for example, an action mode to move the position and posture of the distal end of the endoscope 50 and an action mode to set the zoom magnification, for example.

As illustrated in FIGS. 1 and 2, the remote control apparatus 1 includes operation handles 11, an operation pedal section 12, a display 13, and a control apparatus 14.

The operation handles 11 are provided in order to remotely operate medical equipment (the surgical instruments 4 and the endoscope 50) attached to the robot arms 21 and 22. Specifically, the operation handles 11 accept operations by the operator O for operating the medical equipment. The operation handles 11 include two operation handles 11 arranged side by side in the horizontal direction. One of the two operation handles 11 is operated by the right hand of the operator O while the other operation handle 11 is operated by the left hand of the operator O.

The operation handles 11 extend from the rear side of the remote control apparatus 1 toward the front side. The operation handles 11 are configured to move in a predetermined three-dimensional operation region. Specifically, the operation handles 11 are configured so as to move up and down, right and left, and forward and rearward.

The remote control apparatus 1 and patient-side apparatus 2 constitute a master-slave system in terms of controlling movement of the robot arms 21 and robot arm 22. The operation handles 11 constitute an operating part on the master side in the master-slave system. The robot arms 21 and robot arm 22 holding medical equipment constitute an operating section on the slave side. When the operator O operates the operation handles 11, the movement of the robot arms 21 or 22 is controlled so that the distal end portions (the end effectors 43 of the surgical instruments 4) of the robot arms 21 or the distal end portion (the endoscope 50) of the robot arm 22 moves following the movement of the operation handles 11. In this way, the operations of the robot arms 21 and the robot arm 22 are controlled.

The patient-side apparatus 2 controls the movement of the robot arms 21 in accordance with the set motion scaling ratio. When the motion scaling ratio is set to 1/2, for example, the end effectors 43 of the surgical instruments 4 move 1/2 of the movement distance of the operation handles 11. This allows for precise fine surgery.

The operation pedal section 12 or an operation pedal unit includes plural pedals to execute medical equipment-related functions. The plural pedals include a coagulation pedal, a cutting pedal, a camera pedal, and a clutch pedal. The plural pedals are operated by a foot of the operator O.

The coagulation pedal enables the surgical instrument 4 to coagulate a surgery site. Specifically, when the coagulation pedal is operated, voltage for coagulation is applied to the surgical instrument 4 to coagulate a surgery site. The cutting pedal enables the surgical instrument 4 to cut a surgery site. Specifically, the cutting pedal is operated to apply voltage for cutting to the surgical instrument 4 and cut a surgery site.

The camera pedal is used to control the position and orientation of the endoscope 50 that captures images within the body cavity. Specifically, the camera pedal enables operation of the endoscope 50 by the operation handles 11. That is, the position and orientation of the endoscope 50 are controllable by the operation handles 11 while the camera pedal is being pressed. The endoscope 50 is controlled by using both of the right and left operation handles 11, for example. Specifically, when the operator O rotates the right and left operation handles 11 about the middle point between the right and left operation handles 11, the endoscope 50 is rotated. When the operator O presses the right and left operation handles 11 together, the endoscope 50 goes forward into the body cavity. When the operator O pulls the right and left operation handles 11 together, the endoscope 50 goes back. When the operator O moves the right and left operation handles 11 together up, down, right, or left, the endoscope 50 moves up, down, right, or left, respectively.

The clutch pedal is used to temporarily disconnect operation-related connection between the operation handles 11 and the robot arms 21 and 22 to stop movement of the surgical instruments 4. Specifically, when the clutch pedal is being pressed, the robot arms 21 and 22 of the patient-side apparatus 2 do not work even if the operation handles 11 are operated. For example, when the operation handles 11 are operated and moved to the edge of the range of movement, the operator O operates the clutch pedal to temporarily disconnect the operation-related connection and then returns the operation handles 11 to the center of the range of movement. When the operator O stops operating the clutch pedal, the operation handles 11 are again connected to the robot arms 21 and 22. The operator O restarts the operation for the operation handles 11 around the center thereof.

The display 13 or a display unit is configured to display images captured by the endoscope 50. The display 13 includes a scope type display or a non-scope type display. The scope type display is a display that the operator O looks into. The non-scope type display is a display like an open-type display that includes a flat screen and the operator O is able to see without looking into, such as normal displays for personal computers.

When the scope type display section is attached, the scope type display section displays 3D images captured by the endoscope 50 attached to the robot arm 22 of the patient-side apparatus 2. When the non-scope type display section is attached, the non-scope type display section also displays 3D images captured by the endoscope 50 provided for the patient-side apparatus 2. The non-scope type display section may display 2D images captured by the endoscope 50 provided for the patient-side apparatus.

As illustrated in FIG. 2, the control apparatus 14 includes a controller 141, a storage 142, and an image controller 143, for example. The controller 141 includes a calculator such as a CPU. The storage 142 includes a memory, such as a ROM and a RAM. The control apparatus 14 may be composed of a single controller performing centralized control or may be composed of plural controllers that perform decentralized control in cooperation with each other.

The controller 141 determines whether an action mode instruction inputted by the operation handles 11 is to be executed by the surgical instruments 4 or to be executed by the endoscope 50, depending on the state of the operation pedal section 12. When determining that the action mode instruction inputted by the operation handles 11 is to be executed by any one of the surgical instruments 4, the controller 141 transmits the action mode instruction to the corresponding robot arm 21. The robot arm 21 is thereby driven for controlling movement of the surgical instrument 4 attached to the robot arm 21.

When determining that the action mode instruction inputted by the operation handles 11 is to be executed by the endoscope 50, the controller 141 transmits the action mode instruction to the robot arm 22. The robot arm 22 is thereby driven for controlling movement of the endoscope 50 attached to the robot arm 22.

The storage 142 stores control programs corresponding to the types of the surgical instrument 4, for example. The controller 141 reads the stored control programs according to the types of the attached surgical instruments 4. The action mode instruction from at least one of the operation handles 11 and the operation pedal section 12 of the remote control apparatus 1 thereby causes the respective surgical instruments 4 to perform proper movements.

The image controller 143 transmits images acquired by the endoscope 50 to the display 13. The image controller 143 performs processing and modifying the images when needed.

### (Configurations of Surgical Instrument, Adaptor, Drape, and Robot Arm)

With reference to FIGS. 3 to 6, configurations of the surgical instrument 4, an adaptor 5, a drape 6, and the robot arm 21 are described.

Here, the direction in which the surgical instrument 4 extends (the direction in which the shaft 42 extends) is defined as a Y direction, the distal side (the side toward the end effector 43) of the surgical instrument 4 along the Y direction is defined as a Y1 direction, and the side opposite from the Y1 direction (the proximal side of the surgical instrument 4 (the side toward the housing 41)) is defined as a Y2 direction. The direction in which the surgical instrument 4 and the adaptor 5 are adjacent to each other is defined as a Z direction, the surgical instrument 4 side along the Z direction is defined as a Z1 direction, and the opposite side of the Z1 direction is defined as a Z2 direction. Further, the direction orthogonal to the Y direction and the Z direction is referred to as an X direction, one side along the X direction is referred as an X1 direction, and the other side along the X direction is referred to as an X2 direction. The radial direction of the shaft 42 is referred to as a D direction, and the circumferential direction of the shaft 42 is referred to as an R direction.

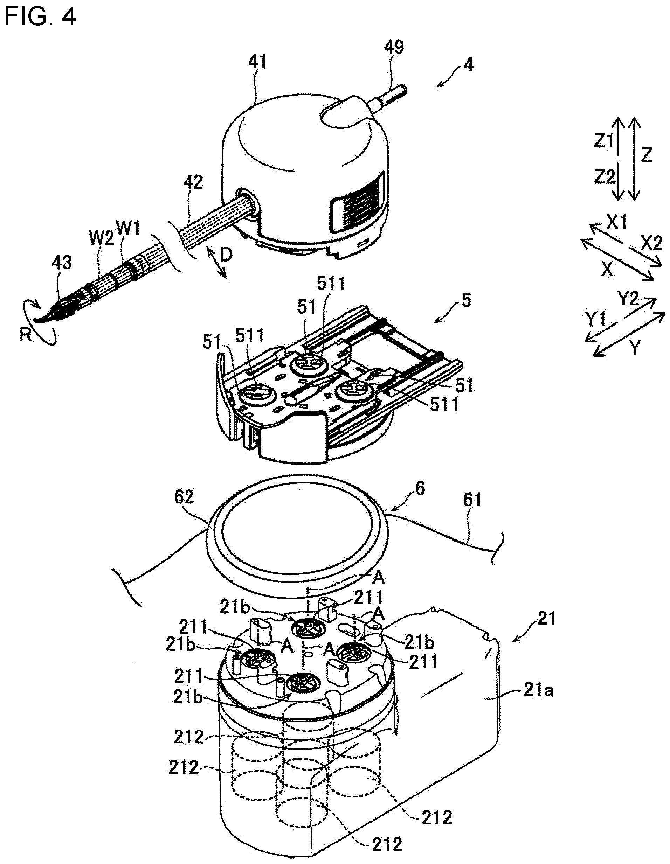

As illustrated in FIGS. 3 and 4, the surgical instrument 4 is detachably connected to the robot arm 21 through the adaptor 5. The adaptor 5 is a drape adaptor configured to sandwich a sterile drape 6 to cover the robot arm 21, in conjunction with the robot arm 21. That is, the adaptor 5 is configured such that the drape 6 is attachable to the adaptor 5.

The surgical instrument 4 is attached to the Z1 side of the adaptor 5. The adaptor 5 is attached to the Z1 side of the robot arm 21.

The robot arm 21 is used in the clean area and is thus covered with the drape 6. In operation rooms, clean technique is used in order to prevent surgical incision sites and medical equipment from being contaminated by pathogen, foreign matters, or the like. The clean technique defines a clean area and a contaminated area, which is outside the clean area. The surgery sites are located in the clean area. Members of the surgical team, including the operator O, make sure that only sterile objects are placed in the clean area during surgery and perform sterilization for an object which is to be moved to the clean area from the contaminated area. Similarly, when an assistant, as one of the members of the surgical team including the operator O, places their hands in the contaminated area, the member sterilize their hands before directly touching objects located in the clean area. Instruments used in the clean area are covered with the drape 6 sterilized or to be sterilized.

As illustrated in FIG. 4, the drape 6 includes a body section 61 that covers the robot arm 21 and an attachment section 62 sandwiched between the robot arm 21 and the adaptor 5. The body section 61 is made of a flexible film member. The flexible film member is made of a resin material, such as thermoplastic polyurethane and polyethylene. The body section 61 includes an opening so that the robot arm 21 is engaged with the adaptor 5. In the opening of the body section 61, the attachment section 62 is provided so as to close the opening. The attachment section 62 is made of a resin mold member. The resin mold member is made of a resin member such as polyethylene terephthalate. The attachment section 62 is harder (less flexible) than the body section 61. The attachment section 62 includes an opening so that the robot arm 21 is engaged with the adaptor 5. The opening of the attachment section 62 may be provided corresponding to the section where the robot arm 21 is engaged with the adaptor 5. The opening of the attachment section 62 may include plural openings corresponding to plural sections at which the robot arm 21 is engaged with the adaptor 5.

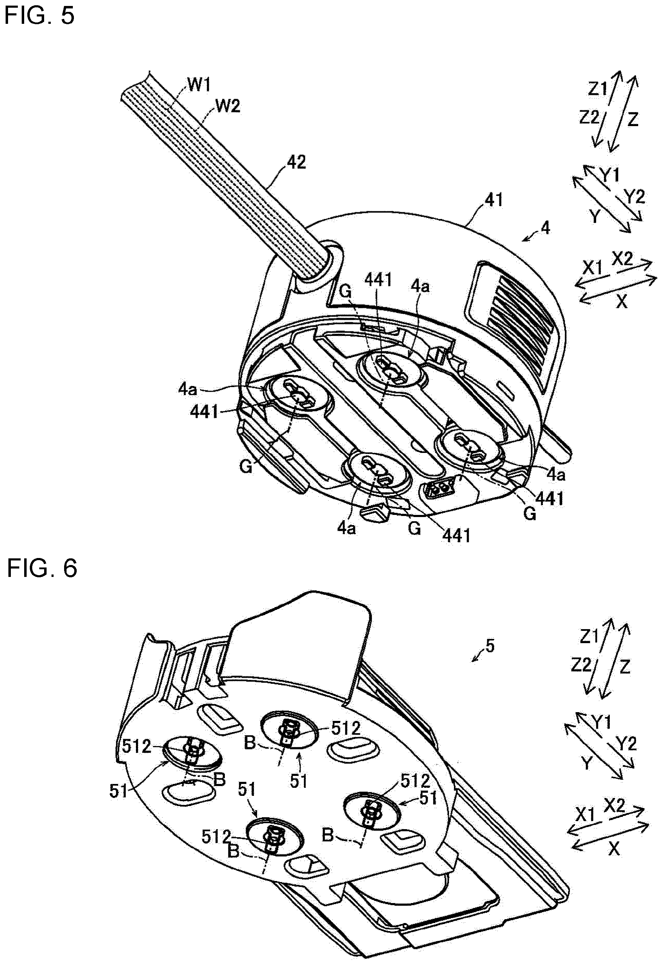

As illustrated in FIGS. 4 and 5, the surgical instrument 4 includes plural (four) driven members 4a. The driven members 4a are provided in the housing 41 and are rotatable about the respective rotation axes G extending along the Z axis. The plural driven members 4a are provided to operate (drive) the end effector 43. For example, one or more of the driven members 4a are connected to the end effector 43 with second elongate elements W2 (described later) inserted through the shaft 42. With this, rotations of the one or more driven members 4a drive the second elongate elements W2, which operate (drive) the end effector 43. In addition, one of the driven members 4a is connected to the shaft 42 through gears (not illustrated), for example. With this, the shaft 42 is rotated with rotation of the one driven member 4a, and the end effector 43 is rotated with rotation of the shaft 42.

To transmit driving forces from the robot arm 21 to the end effector 43, the driven members 4a respectively include engagement projections 441, which are engaged with later-described drive transmission members 51 of the adaptor 5. The engagement projection 441 is projected from the Z2 side surface of each driven member 4a toward the side of the adaptor 5 (the Z2 side). The engagement projection 441 has a shape corresponding to an engagement recess 511 (see FIG. 4) of the corresponding drive transmission member 51 of the adaptor 5 and having projected portions arranged in line. The engagement projection 441 has a line-symmetric shape.

As illustrated in FIGS. 4 and 6, the adaptor 5 includes a plurality (four) of the drive transmission members 51. The drive transmission members 51 are configured to transmit driving forces from the robot arm 21 to the driven members 4a of the surgical instrument 4. That is, the drive transmission members 51 are provided so as to correspond to the driven members 4a of the surgical instrument 4. The drive transmission members 51 are rotatable about the respective rotation axes B, which extend along the Z direction.

As illustrated in FIG. 4, each of drive transmission members 51 includes the engagement recess 511 which is respectively engaged with the engagement projection 441 of the corresponding driven member 4a of the surgical instrument 4. The engagement recess 511 is located at the surgical instrument 4 side (the Z1 side) of the drive transmission member 51 and is recessed from the Z1 side surface of the drive transmission member 51, toward the Z2 direction, opposite to the surgical instrument 4. Each of the engagement recesses 511 has a line-symmetric shape.

As illustrated in FIG. 6, each of the drive transmission members 51 includes an engagement recess 512, which is engaged with an engagement projection 211 of the corresponding drive part 21b of the robot arm 21. The engagement recess 512 is located at the robot arm 21 side (the Z2 side) of the drive transmission member 51. The engagement recess 512 is recessed from the Z2 side surface of the drive transmission member 51, toward the Z1 direction, opposite to the robot arm 21. The plural drive transmission members 51 include substantially the same configuration. Each of the engagement recesses 512 has a line-symmetric shape.

As illustrated in FIG. 4, the robot arm 21 includes a frame 21a and the plural (four) drive parts 21b. Each of the drive parts 21b is attached to the frame 21a of the robot arm 21. The plural drive parts 21b are provided corresponding to the plural (four) drive transmission members 51 of the adaptor 5. Each of the drive parts 21b has the same or a similar configuration, and thus only one of the drive parts 21b is described below to avoid redundancy.

Each of the drive parts 21b includes the engagement projection 211 and an actuator 212.

The engagement projection 211 of each drive part 21b is engaged with the engagement recess 512 of the corresponding drive transmission member 51 (see FIG. 6). Each of the engagement projections 211 is projected from the Z1 side surface of the drive part 21b toward the Z1 side (the adaptor 5 side). Each of the engagement projections 211 has a line-symmetric shape.

The actuator 212 includes a motor. The actuator 212 is configured to drive the engagement projection 211 to rotate about the rotational axis A extending in the Z direction. Thereby, the drive transmission member 51 of the adaptor 5 engaged with the engagement projection 211 can be rotated about the rotational axis B extending in the Z direction, and the driven member 4a of the surgical instrument 4 engaged with the drive transmission member 51 can be rotated about the rotational axis G. Note that the rotational axis A, the rotational axis B, and the rotational axis G are coaxially arranged.

### (Detailed Explanation of Surgical Instrument)

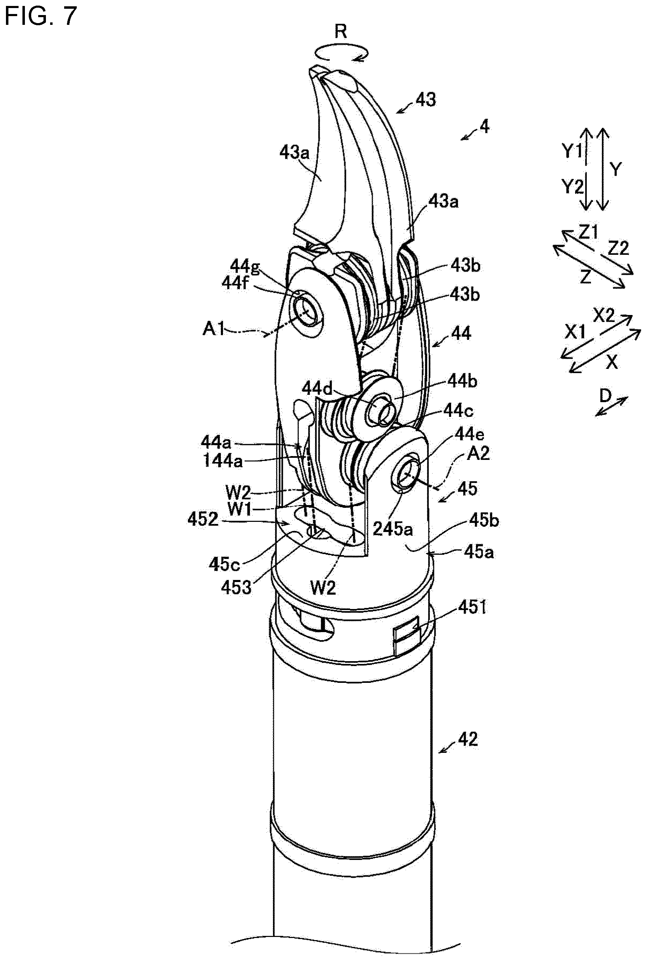

With reference to FIGS. 7 to 16, the surgical instrument 40 is described in detail. The surgical instrument 4 according to a first embodiment is configured to have a seal member 246 (described later) that seals the inside of the shaft 42 to prevent gas (such as carbon dioxide, etc.) filled in the body cavity of the patient for the surgery in an abdominal cavity or the like from leaking out of the treatment site where the surgical instrument 4 is inserted. That is, the surgical instrument 4 is configured to prevent the gas filled in the body cavity of the patient from leaking into the surgical instrument 4 and thus prevent the gas from passing through the surgical instrument 4 to the outside of the body of the patient.

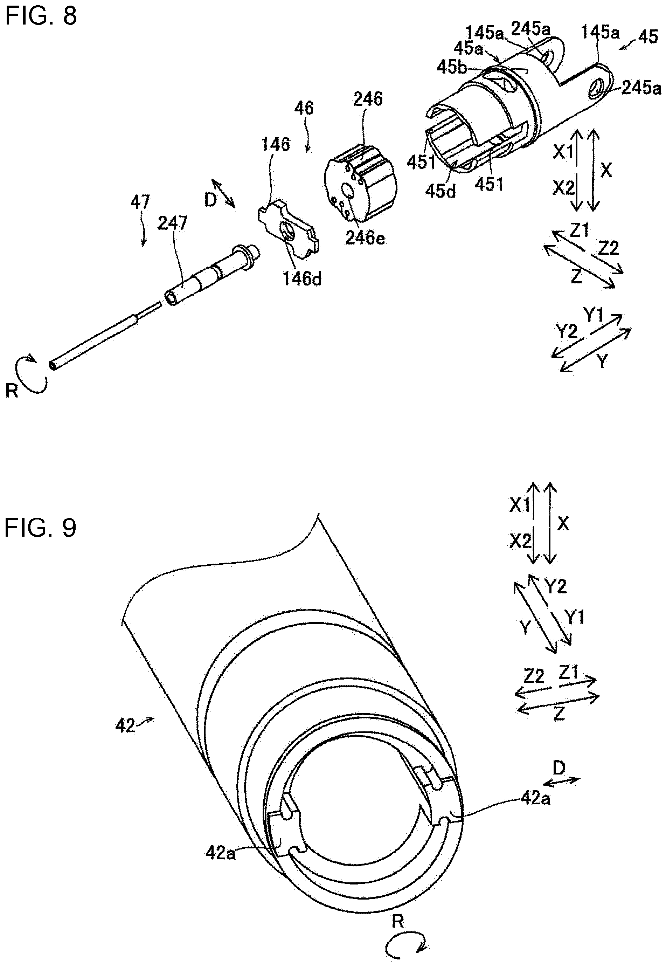

As illustrated in FIGS. 7 and 8, the surgical instrument 4 is, for example, an electrosurgical instrument such as a monopolar curved scissors. The surgical instrument 4 includes the conductive end effector 43, a conductive distal clevis 44 that rotatably supports the end effector 43 about a first axis A1, and a conductive proximal clevis 45 that rotatably supports the distal clevis 44 about a second axis A2. The surgical instrument 4 includes a cylindrical tubular shaft 42 connected to the proximal clevis 45, and a sealing mechanism 46 (a sealing device 46) provided in contact with the proximal clevis 45 between the shaft 42 and the proximal clevis 45 and including at least a part thereof having conductivity. The surgical instrument 4 includes an electric wire 47. The electric wire (conductor) 47 includes an end effector-side end portion (a distal end portion) connected to the sealing mechanism 46 so as to supply electrical energy to the end effector 43 through the sealing mechanism 46, the proximal clevis 45, and the distal clevis 44. The distal clevis 44 is an example of a distal support body and the proximal clevis 45 is an example of a proximal support body.

The surgical instrument 4 includes the sealing mechanism 46 that is in contact with the proximal clevis 45 and includes at least a part thereof having conductivity, and the electric wire 47 that includes the end effector-side end portion (the Y1-side end portion) connected to the sealing mechanism 46. With this, electricity can flow from the electric wire 47 to the end effector 43 via the sealing mechanism 46, the proximal clevis 45, and the distal clevis 44. Here, the surgical instrument 4 is configured such that the electric wire 47 is ended at the sealing mechanism 46 and thus does not penetrate through the sealing mechanism 46. As a result, the sealing performance of the sealing mechanism 46 can be improved.

As illustrated in FIG. 7, the end effector 43 includes plural (two) end effector members 43a. Each of the end effector members 43a is, for example, a jaw member made of a conductive material such as stainless steel or the like.

Each of the end effector members 43a includes a pulley portion 43b. Each of the end effector members 43a is configured to change its posture as a second elongate element W2 wound around the pulley portion 43b thereof moves. Specifically, a circular column attachment (fastener) attached to the second elongated element W2 is engaged with the pulley portion 43b of each end effector member 43a. As the second elongated element W2 moves, the pulley portion 43b rotates about the first axis A1 thereof, and thus the end effector member 43a rotates about the first axis A1. Here, the case has been described in which the end effector 43 is a pair of scissors; however, the end effector 43 may be an end effector other than a pair of scissors. Also, the end effector 43 may be composed of a single end effector member 43a. For example, the end effector 43 may be a hook, a spatula, or the like.

The distal clevis 44 includes a pully portion 44a, a first pulley group 44b (first pulleys 44b), a second pulley group 44c (second pulleys 44b), a first shaft portion 44d, a second shaft portion 44e, and a third shaft portion 44f. Note that the second shaft portion 44e is an example of a shaft portion.

At the distal end side (the end effector 43 side) of the distal clevis 44, a pair of shaft holes 44g are formed. The third shaft portion 44f, which rotatably supports the pulley portions 43b of the end effector members 43a, is inserted in the pair of shaft holes 44g. The third shaft portion 44f is a conductive shaft member formed in a circular column shape extending along the first axis A1. The third shaft portion 44f is supported by the pair of shaft holes 44g. The first axis A1 extends along the direction substantially orthogonal to the Y direction.

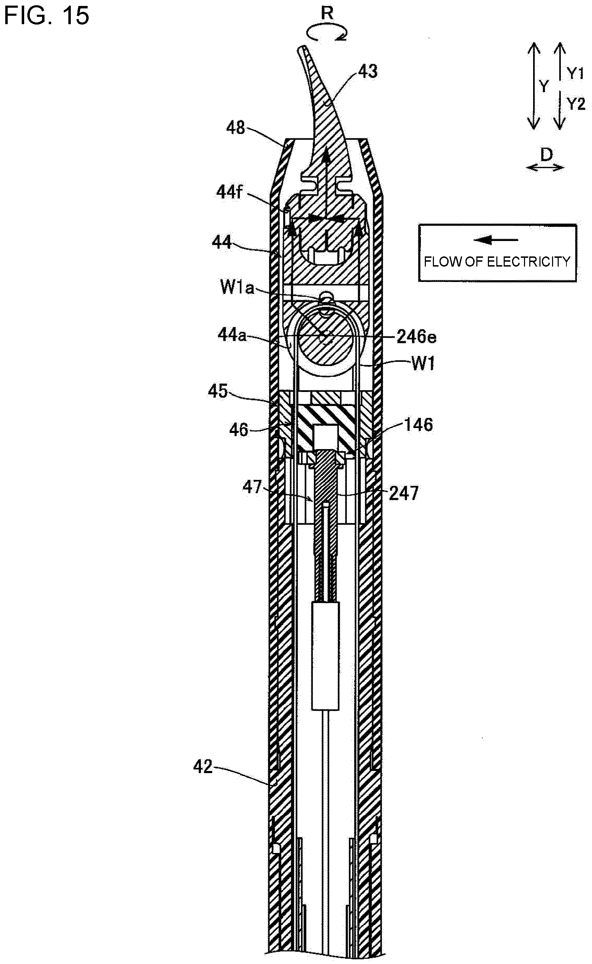

The pulley portion 44a is provided on the proximal end side (the shaft 42 side) of the distal clevis 44 and is rotatably supported by the proximal clevis 45 about the second axis A2. Specifically, the pulley portion 44a is rotatably supported by the second shaft portion 44e which is supported by the proximal clevis 45. The second axis A2 extends along the direction substantially orthogonal to the Y direction and substantially orthogonal to the direction parallel to the first axis A1. The pulley portion 44a includes a pulley groove formed along a circumferential direction of the second axis A2. The distal clevis 44 is configured to change its posture as a first elongate element W1 wound around the pulley portion 44a thereof moves. Specifically, a circular column-shaped attachment (fastener) W1a (see FIG. 15) attached to the first elongate element W1 is engaged with the pulley portion 44a. As the second elongate element W2 moves, the pulley portion 44a rotates about the second rotational axis A2 and thus the distal clevis 44 rotates about the second rotational axis A2.

The first pulley group 44b (first pulleys 44b) is rotatably supported by the first shaft portion 44d. The second pulley group 44c (second pulleys 44c) is rotatably supported by the second shaft portion 44e. The first pulley group 44b and the second pulley group 44c guide the second elongate elements W2 engaged with the pulley portions 43b of the end effector members 43a. The first pulley group 44b is arranged between the second axis A2 and the first axis A1. The second pulley group 44c is arranged on the second axis A2.

The first shaft portion 44d extends along a rotation center line (axis) substantially parallel to the second axis A2. The second shaft portion 44e is a conductive shaft member formed in a circular column shape extending along the second axis A2. The second shaft portion 44e is inserted in and supported by a pair of shaft holes 245a (described later) of the proximal clevis 45.

Here, each of the first elongate element W1 and the second elongate elements W2 is a wire or a cable. Each of the first elongate element W1 and the second elongate elements W2 is made of a metal such as stainless steel, tungsten, or the like. The number of the first elongate element(s) W1 is set corresponding to the number of the pulley portion(s) 44a. In this example, the number of the first elongate element W1 is one. The number of the second elongate element(s) W2 is set corresponding to the number of the end effector member(s) 43a. In this example, the number of the second elongate elements W2 is two. Note that the first elongate element W1 and the second elongate elements W2 may be rods or the like.

As illustrated in FIGS. 7 and 8, the proximal clevis 45 includes a connection base 45a to be connected to the shaft 42.

The connection base 45a is formed in a substantially cylindrical shape. The connection base 45a is connected to the shaft 42. Specifically, a Y1-side end portion of the shaft 42 is connected to a Y2-side end portion of the connection base 45a. The connection base 45a includes a circumferential wall portion 45b extending along the R direction and a partition wall portion 452 provided in an end effector-side end portion of the connection base 45a (a Y1-side end portion of the connection base 45a). The circumferential wall portion 45b of the proximal clevis 45 is formed with cutouts 451 (or notches 451) recessed from the shaft 42 side end (the proximal end) of the circumferential wall portion 45b toward the end effector 43 side (toward the distal side) in the Y direction (the axial direction of the shaft 42). A plurality (two) of cutouts 451 are provided at positions facing each other in the D direction so as to correspond to projections 42a (described later) of the shaft 42.

The connection base 45a includes a pair of support portions 145a which rotatably support the distal clevis 44. The pair of support portions 145a are opposed to each other in the Z direction. Each support portions 145a protrudes in the Y1 direction (the distal direction) from the Y1-side end portion of the connection base 45a. Each support portion 145a is formed with a shaft hole 245a in which the second shaft portion 44e is inserted. The second shaft portion 44e is a conductive shaft member formed in a circular column shape extending along the second axis A2 and is supported by the pair of shaft holes 245a.

The connection base 45a includes an inner space 45d surrounded by the circumferential wall portion 45b and the partition wall portion 452. The inner space 45d of the connection base 45a is opened in the Y2 direction (the proximal direction).

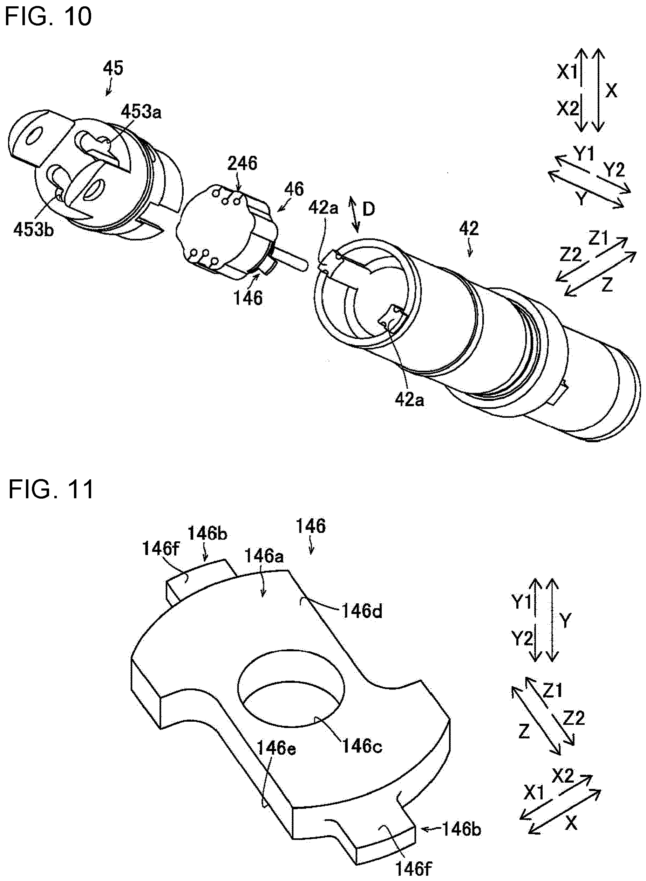

The partition wall portion 452 is configured to separate the inner space 45d and the outer space of the connection base 45a. The partition wall portion 452 is formed with communication holes 453 that connect the inner space 45d of the connection base 45a and the outer space of the connection base 45a. Here, the communication holes 453 formed in the partition wall portion 452 include a communication hole 453 located on the X1 side (hereinafter, a first communication hole 453a (see FIG. 10)) and a communication hole 453 located on the X2 side (hereinafter, a second communication hole 453b (see FIG. 10)). The first communication hole 453a and the second communication hole 453b are formed in a substantial T-shape as seen in the Y direction.

As illustrated in FIGS. 9 and 10, the shaft 42 is formed in a cylindrical tubular shape extending along the Y direction and is made of an insulating material such as epoxy resin, or the like, for example. The first elongate element W1 and the second elongate elements W2 are housed in the space inside the shaft 42. The shaft 42 includes the projections 42a projecting from the Y1 side end (the distal end) of the shaft 42 toward the Y1 side (the end effector 43 side, the distal side). A plurality (two) of the projections 42a are provided at positions facing each other in the D direction. The projections 42a are respectively inserted in the corresponding cutouts 451 of the proximal clevis 45.

### (Sealing mechanism)

The sealing mechanism 46 (the sealing device 46) includes a retainer 146 and the seal member 246 that seals the inner space of the shaft 42. The seal member 246 is an example of a seal member. The retainer 146 is an example of a retaining member.

### (Retainer)

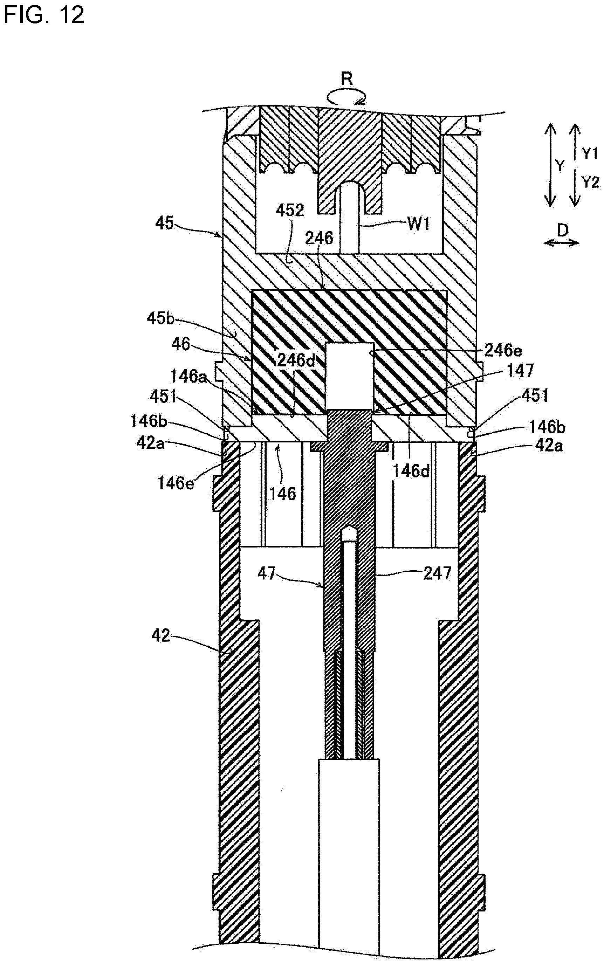

As illustrated in FIGS. 11 and 12, the retainer 146 includes a pressing surface 146d that is in contact with a shaft 42 side surface 246d (a Y2-side surface, a distal side surface) of the seal member 246 and presses the shaft-side surface 246d in the D direction in an area except for a center portion of the surface 246d.

The retainer 146 is configured to suppress unevenness in force pressing the shaft-side surface 246d of the seal member 246. That is, the retainer 146 is configured to apply the pressing force from the shaft 42 substantially uniformly to the shaft-side surface 246d of the seal member 246. The retainer 146 is, for example, made of a metal such as stainless steel or the like and has conductivity. Note that the retainer 146 is not limited to stainless steel, and may be formed of a conductive material other stainless steel, for example.

Specifically, the retainer 146 includes a main body 146a having a substantially plate-shape, pressed portions 146b, and a through hole 146c. The main body 146a includes the pressing surface 146d provided on the Y1 side (the end effector 43 side) and a pressed surface 146e provided on the Y2 side (the shaft 42 side). Here, the pressing surface 146d is an example of a first surface and the pressed surface 146e is an example of a second surface.

The pressing surface 146d is formed of a surface of the retainer 146 on the distal side and is in contact with the shaft-side surface 246d (the proximal-side surface) of the seal member 246. Specifically, the pressing surface 146d extends in the D direction and is in contact with the shaft-side surface 246d of the seal member 246 in an area except for a center portion of the shaft-side surface 246d in the D direction, so that the pressing surface 146d is pressed against the shaft-side surface 246d of the seal member 246.

The pressed surface 146e is formed of a surface of the retainer 146 on the proximal side and is arranged on the Y2 side (the proximal side) with respect to the pressing surface 146d.

A plurality (two) of the pressed portions 146b are provided corresponding to the projections 42a of the shaft 42. Each of the pressed portions 146b is configured to be pressed by the corresponding projection 42a of the shaft 42. The two pressed portions 146b are provided line-symmetrically with respect to the main body 146a. Specifically, the two pressed portions 146b are respectively projected from an edge portion of the main body 146a outwardly in the D direction. The surface of each pressed portion 146b on the shaft 42 side (the Y2 side, the proximal side) is flush with the pressed surface 146e.

The through hole 146c of the retainer 146 penetrates through the retainer 146 in the Y direction. The through hole 146c is provided at a center portion of the retainer 146 in the D direction. The through hole 146c has a size in which a tip portion (a Y1-side end portion, a distal end portion) of a connector portion 247 (described later) of the electric wire 47 can be inserted.

### (Seal Member)

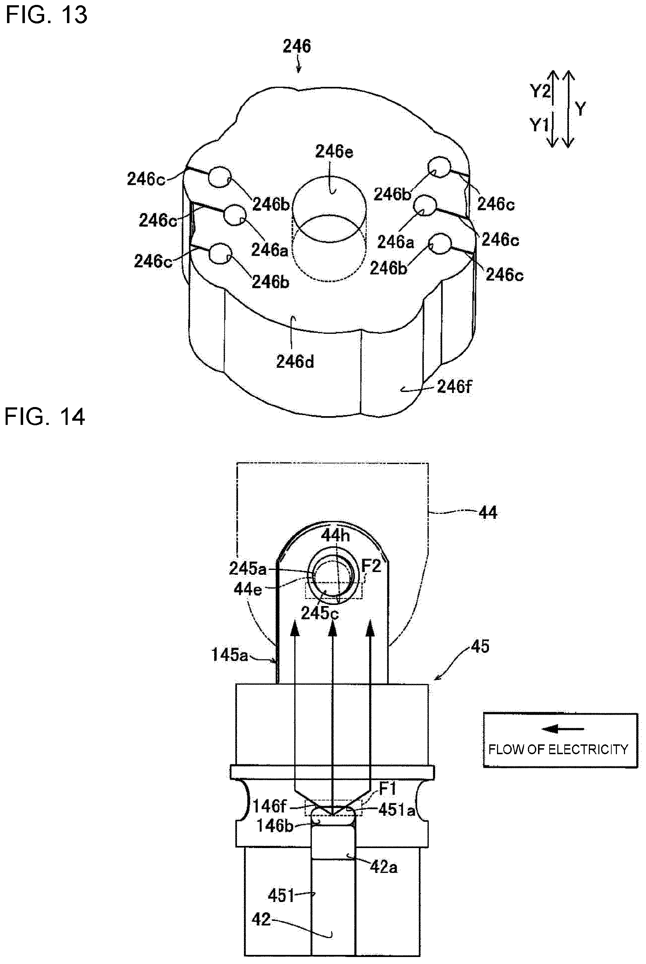

As illustrated in FIGS. 12 and 13, the seal member 246 is disposed in the proximal clevis 45 and is configured to prevent the gas filled in the body cavity of the patient P from leaking out of the body from the treatment site via the shaft 42.

Specifically, the seal member 246 is made of an elastically deformable insulating material. The seal member 246 is, for example, made of an elastomer such as silicone rubber or the like. The seal member 246 has a thickness in the Y direction. The seal member 246 is compressed at a Y1-side end (a distal side end) of the inner space 45d of the proximal clevis 45. The seal member 246 is in close contact with the circumferential wall portion 45b and the partition wall portion 452 of the proximal clevis 45.

The seal member 246 closes (covers) the first communication hole 453a and the second communication hole 453b of the proximal clevis 45. Further, the seal member 246 is configured such that the first elongate element W1 and the second elongate elements W2 can be attached to the seal member 246, in a state where the first elongate element W1 is attached to the distal clevis 44 and the second elongate elements W2 are attached to the end effector 43.

Specifically, the seal member 246 includes first insertion holes 246a, second insertion holes 246b, and slits 246c. In the first insertion holes 246a, the first elongate element W1 is inserted. The first insertion holes 246a penetrate through the seal member 246 in the Y direction. A plurality (two, in this example) of the first insertion holes 246a are provided in the seal member 246. In the second insertion holes 246b, the second elongate elements W2 are inserted. The second insertion holes 246b penetrate through the seal member 246 in the Y direction. A plurality (four, in this example) of the second insertion holes 246b are provided in the seal member 246.

The slits 246c respectively extend (connect) from the first insertion holes 246a and the second insertion holes 246b to an outer circumferential surface 246f of the seal member 246.

The Y2-side surface 246d (the proximal side surface) of the seal member 246, which is, the shaft 42 side surface of the seal member 246, is formed with an accommodation recess 246e.

The accommodation recess 246e is a concave portion recessed from the Y2-side surface 246d (the proximal side surface, the shaft 42 side surface) of the seal member 246 toward the Y1 side (the distal side, the end effector 43 side). That is, the accommodation recess 246e does not penetrate through the seal member 246. The accommodation recess 246e has a substantially circular shape as seen from the Y2 side. The diameter of the accommodation recess 246e is larger than that of the tip portion of the connector portion 247 of the electric wire 47 as seen from the Y2 side. The accommodation recess 246e is arranged on a rotational center line (a rotational axis) of the shaft 42. The accommodation recess 246e is coaxially arranged with the electric wire 47. The number of the accommodation recess 246e is one and the accommodation recess 246e is formed at the center of the Y2-side surface 246d of the seal member 246. As will be described in detail later, the accommodation recess 246e is an escape portion for avoiding contact between the tip portion of the connector portion 247 of the electric wire 47 and the seal member 246.

### (Electric Wire)

As illustrated in FIG. 12, an end effector-side end portion 147 (a Y1-side end portion, a distal end portion) of the electric wire 47 is formed with the connector portion 247, which is to be connected to the sealing mechanism 46.

With this configuration, the electric wire 47 and the sealing mechanism 46 can be reliably connected by the connector portion 247. Thus, electrical energy can be stably supplied from the electric wire 47 to the end effector 43.

The tip portion (the Y1-side end portion, the distal end portion) of the connector portion 247 of the electric wire 47 is inserted in the through hole 146c of the retainer 146 and caulked and fixed in the through hole 146c. That is, the distal end portion 147 (the end effector-side end portion) of the electric wire 47 is terminated at the retainer 146.

With this, the connector portion 247 of the electric wire 47 can be easily fixed to the retainer 146 in a state where the connector portion 247 is in close contact with the retainer 146. Therefore, it is possible to obtain the surgical instrument 4 in which the electric wire 47 and the retainer 146 are securely electrically connected.

The tip portion of the connector portion 247 of the electric wire 47 is accommodated in the accommodation recess 246e of the seal member 246 without coming into contact with the seal member 246. In other words, the accommodation recess 246e is provided in the seal member 246 so as to avoid (so as not to interfere with) the connector portion 247 of the electric wire 47. Here, the accommodation recess 246e is not penetrated through the seal member 246, that is, the sealing mechanism 46 does not have a through hole for passing the electric wire 47.

With this configuration, the tip portion of the connector portion 247 of the electric wire 47 does not press the seal member 246. Therefore, it is possible to prevent formation of a gap between the Y2-side surface 246d (the shaft-side surface 246d) of the seal member 246 and the pressing surface 146d of the retainer 146. As a result, since the seal member 246 can be reliably pressed by the retainer 146, the sealing performance of the seal member 246 can be improved.

Note that the Y2-side end portion (the proximal end portion) of the electric wire 47 is connected to an electrode 49 that is attached to the housing 41 (see FIG. 3). The electrode 49 is connected to a generator (not illustrated) of the robotic surgical system 100 via a power supply cable.

### (Electrical Connection)

Here, an electrical connection from the electric wire 47 to the end effector 43 in the surgical instrument 4 is described.

The sealing mechanism 46 includes the seal member 246 that is provided between the proximal clevis 45 and the shaft 42 and the conductive retainer 146 that is pressed by the shaft 42 to press the shaft-side surface 246d of the seal member 246. The electric wire 47 is connected to the retainer 146 at the connector portion 247 of the electric wire 47, and thus supplies the electrical energy to the end effector 43 through the retainer 146, the proximal clevis 45, and the distal clevis 44.

With this configuration, since the electric wire 47 is connected to the retainer 146 which presses the seal member 246 from the shaft 42 side and is in contact with the proximal clevis 45, the electric wire 47 can be electrically connected to the proximal clevis 45, the distal clevis 44, and the end effector 43 through the retainer 146, without forming a through hole for passing the electric wire 47 in the seal member 246. Consequently, the sealing performance of the seal member 246 can be improved. Further, with this configuration, after the seal member 246 is disposed, the retainer 146 that is connected to the electric wire 47 can be disposed. Accordingly, the assembly of the seal member 246 to the proximal clevis 45 can be easily performed.

Specifically, the electric wire 47 and the retainer 146 are electrically connected. As described above, the end effector-side end portion of the connector portion 247 is caulked in the through hole 146c. With this, the connector portion 247 electrically connects the electric wire 47 and the conductive retainer 146.

As illustrated in FIG. 14, the retainer 146 and the proximal clevis 45 are electrically connected.

Specifically, the proximal clevis 45 includes the cutouts 451 recessed from the shaft 42 side end of the proximal clevis 45 toward the end effector 43 side in the Y direction (the axial direction of the shaft 42). The retainer 146 includes the pressed portions 146b arranged in the cutouts 451 of the proximal clevis 45 and configured to be pressed by the shaft 42. In the state where the pressed portions 146b of the retainer 146 are pressed by the shaft 42, inner surfaces 451a of the cutouts 451 are in contact with the pressed portions 146b. With this, the electric wire 47 is electrically connected to the proximal clevis 45 via the retainer 146.

With this configuration, by the shaft 42 pressing the retainer 146, the pressed portions 146b of the retainer 146 and the inner surfaces 451a of the cutouts 451 can be firmly in contact with each other. Therefore, the electric wire 47 and the proximal clevis 45 can be reliably electrically connected via the retainer 146.

Here, the inner surface 451a (see FIG. 14) of each cutout 451 on the end effector 43 side has a shape that allows surface contact with an end effector 43 side surface 146f of the corresponding pressed portion 146b. For example, the inner surface 451a of the cutout 451 on the end effector 43 side and the end effector 43 side surface 146f of the pressed portion 146b respectively include flat surface areas extending in a direction (X direction, D direction) orthogonal to the axial direction (Y direction) of the shaft 42, and these flat surface areas are in surface contact with each other.

As a result, a sufficient contact area between the retainer 146 and the proximal clevis 45 can be secured, so that electrical energy can be stably supplied from the electric wire 47 to the end effector 43 via the retainer 146 and the proximal clevis 45.

Here, the inner surface 451a of the cutout 451 on the end effector 43 side is a Y1-side end area of the inner circumferential surface of the cutout 451. The pressed portion 146b is formed in a substantially rectangular shape as seen in the D direction. The end effector 43 side surface 146f of the pressed portion 146b is a surface of the pressed portion 146b on the Y1 side. The inner surface 451a of the cutout 451 on the end effector 43 side has the shape that corresponds to the end effector 43 side surface 146f of the pressed portion 146b. Thus, the inner surface 451a of the cutout 451 on the end effector 43 side has the shape substantially same as that of the end effector 43 side surface 146f of the pressed portion 146b. That is, the inner surface 451a of the cutout 451 on the end effector 43 side and the end effector 43 side surface 146f of the pressed portion 146b respectively include flat surface areas extending in the direction (X direction, D direction) orthogonal to the axial direction (Y direction) of the shaft 42, and these flat surface areas are in surface contact with each other. Note that, in a modification, the inner surface 451a of the cutout 451 on the end effector 43 side and the end effector 43 side surface 146f of the pressed portion 146b may not include flat surface areas extending in the direction (X direction) orthogonal to the axial direction (Y direction) of the shaft 42.

The pressed portions 146b of the retainer 146 are pressed by the projections 42a of the shaft 42 from the Y2 side toward the Y1 side, and therefore the inner surfaces 451a of the cutouts 451 on the end effector 43 side and the end effector 43 side surfaces 146f of the pressed portions 146b are brought in close contact with each other (see the FI portion in FIG. 14). Accordingly, the electricity is flown from the retainer 146 to the proximal clevis 45 (see the arrows in FIG. 14.)

As illustrated in FIGS. 14 and 15, the proximal clevis 45 and the distal clevis 44 are electrically connected.

Specifically, the surgical instrument 4 includes the first elongate element W1 for rotationally driving the distal clevis 44 about the second shaft portion 44e with respect to the proximal clevis 45. The proximal clevis 45 includes the pair of shaft holes 245a supporting the second shaft portion 44e. In a state where the distal clevis 44 is pulled by the first elongate element W1 toward the shaft 42 side, an inner surface 245c of the shaft hole 245a on the shaft 42 side and a surface 44h of the second shaft portion 44e on the shaft 42 side are in contact with each other, so that the proximal clevis 45 is electrically connected to the end effector 43 via the distal clevis 44.

With this configuration, the proximal clevis 45 and the distal clevis 44 can be brought into contact with each other by utilizing the tension of the first elongated element W1. Accordingly, it is possible to suppress increase in the number of parts of the surgical instrument 4 as compared with a case where the proximal clevis 45 and the distal clevis 44 are brought into contact with each other by a dedicated structure.

The shaft hole 245a has a substantially circular shape as seen in the D direction (X direction). The inner surface 245c of the shaft hole 245a on the shaft 42 side is a portion of the inner circumferential surface of the shaft hole 245a on the Y2 side with respect to the center of the shaft hole 245a in the Y direction. The inner surface 245c of the shaft hole 245a on the shaft 42 side has an arc shape as seen in the D direction. The second shaft portion 44e has a substantially circular shape as seen in the D direction. The surface 44h of the second shaft portion 44e on the shaft 42 side is a portion of the outer circumferential surface of the second shaft portion 44e on the Y2 side with respect to the center of the second shaft portion 44e in the Y direction. The surface 44h of the second shaft portion 44e on the shaft 42 side has an arc shape as seen in the D direction. Accordingly, the inner surface 245c of the shaft hole 245a on the shaft 42 side has a shape substantially same as that of the surface 44h of the second shaft portion 44e on the shaft 42 side.

By the first elongate element W1 pulling the pulley portion 44a from the Y1 side toward the Y2 side, the inner surface 245c of the shaft hole 245a on the shaft 42 side and the surface 44h of the second shaft portion 44e on the shaft 42 side are firmly in close contact with each other (see the F2 portion of FIG. 14). Accordingly, the electricity is flown from the proximal clevis 45 to the distal clevis 44 (see the arrows in FIG. 15).

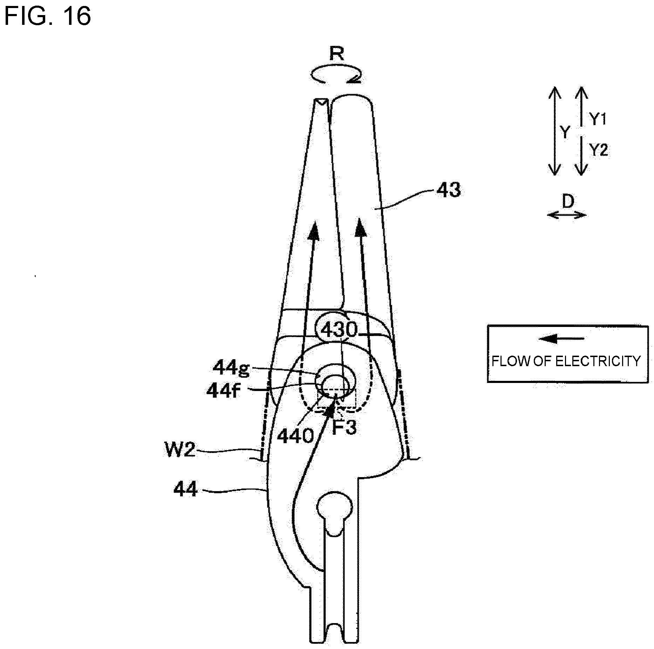

As illustrated in FIGS. 15 and 16, the distal clevis 44 and the end effector 43 are electrically connected.

More specifically, in a state where the end effector 43 is pulled by the second elongate element W2 toward the shaft 42 side, a surface 430 of the third shaft portion 44f on the shaft 42 side and an inner surface 440 of the shaft hole 44g of the distal clevis 44 on the shaft 42 side are in contact with each other, so that the proximal clevis 45 and the end effector 43 are electrically connected to each other via the distal clevis 44.

Here, the third shaft portion 44f has a substantially circular shape as seen in the D direction (the radial direction of the shaft 42, the X direction). The surface 430 of the third shaft portion 44f on the shaft 42 side is a portion of the outer circumferential surface of the third shaft portion 44f on the Y2 side with respect to the center of the third shaft portion 44f in the Y direction. The surface 430 of the third shaft portion 44f on the shaft 42 side has an arc shape as seen in the D direction. The shaft hole 44g of the distal clevis 44 has a substantially circular shape as seen in the D direction (X direction). The inner surface 440 of the shaft hole 44g of the distal clevis 44 on the shaft 42 side is a portion of the inner circumferential surface of the shaft hole 44g of the distal clevis 44 on the Y2 side with respect to the center of the shaft hole 44g in the Y direction. The inner surface 440 of the shaft hole 44g of the distal clevis 44 on the shaft 42 side has an arc shape as seen in the D direction. Accordingly, the inner surface 440 of the shaft hole 44g of the distal clevis 44 on the shaft 42 side has a shape substantially same as that of the surface 430 of the third shaft portion 44f on the shaft 42 side.

By the second elongate elements W2 pulling the pulley portion 43b of the end effector 43 from the Y1 side toward the Y2 side, the surface 430 of the third shaft portion 44f on the shaft 42 side and the inner surface 440 of the shaft hole 44g of the distal clevis 44 on the shaft 42 side are firmly in close contact with each other (see the F3 portion in FIG. 16). Accordingly, the electricity is flown from the proximal clevis 45 to the distal clevis 44 via the third shaft portion 44f (see the arrows in FIGS. 15 and 16).

### (Insulating Cover)

As illustrated in FIGS. 3 and 15, the surgical instrument 4 includes an insulating cover 48 that covers the conductive distal clevis 44, the conductive proximal clevis 45, and the sealing mechanism 46 having conductivity from the outside in the D direction (X direction). Note that FIGS. 4 and 7 illustrate the surgical instrument 4 with the insulating cover 48 removed (omitted).

With this configuration, it is possible to prevent the electrical energy from transmitting from the distal clevis 44, the proximal clevis 45, and the retainer 146 of the sealing mechanism 46 to the outside of the surgical instrument.

Specifically, the cover 48 has a substantially truncated cone shape having a cylindrical main body portion and a tapered tip portion. The cover 48 is an insulating member. The cover 48 is made of an elastomer such as silicone rubber or the like. The cover 48 is configured to be elastically deformable. The cover 48 covers a Y1-side portion of the shaft 42, the proximal clevis 45, the distal clevis 44, and the pulley portion 43b of the end effector 43.

The surgical instrument 4 as described above is a monopolar electrosurgical instrument including the single electric wire 47.

With this configuration, the monopolar electrosurgical instrument can improve the sealing performance of the sealing mechanism 46.

### [Second Embodiment]

With reference to FIG, 17, a configuration of a surgical instrument 604 according to a second embodiment is described. In a second embodiment, unlike a first embodiment, a seal member 646a has conductivity. In a second embodiment, descriptions of the same configurations as those in a first embodiment may be omitted.

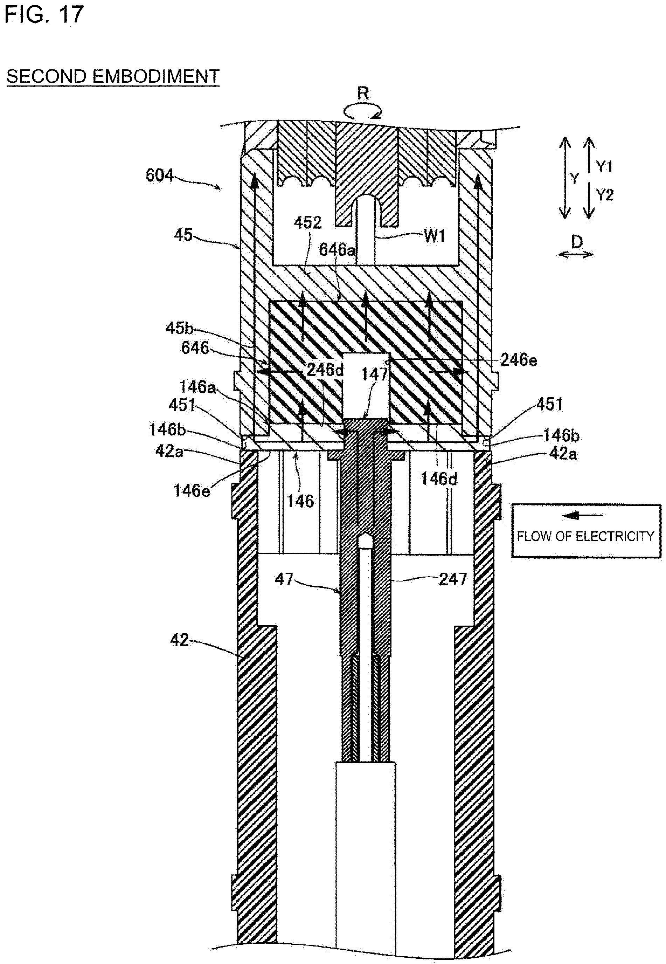

As illustrated in FIG. 17, the surgical instrument 604 according a second embodiment includes the end effector 43, the conductive distal clevis 44 supporting the end effector 43 to be rotatable about the first axis A1, and the conductive proximal clevis 45 supporting the distal clevis 44 to be rotatable about the second axis A2. The surgical instrument 604 includes the cylindrical tubular shaft 42 to be connected to the proximal clevis 45, and a sealing mechanism 646 (a sealing device 646) provided in contact with the proximal clevis 45 and including at least a part thereof having conductivity. The surgical instrument 604 includes the electric wire 47. The electric wire 47 includes the end effector-side end portion thereof connected to the sealing mechanism 646 to supply the electrical energy to the end effector 43 through the sealing mechanism 646, the proximal clevis 45, and the distal clevis 44.

With this configuration, like a first embodiment, the sealing performance of the sealing mechanism 646 of the surgical instrument 604 can be improved.

Here, the direction in which the surgical instrument 604 extends (the direction in which the shaft 42 extends) is defined as the Y direction, the distal side (the side toward the end effector 43) of the surgical instrument 604 along the Y direction is defined as the Y1 side, and the opposite side of the Y1 side is defined as the Y2 side. The radial direction of the shaft 42 is referred to as the D direction, and the circumferential direction of the shaft 42 is referred to as the R direction.

### (Sealing mechanism)

The sealing mechanism 646 includes the retainer 146 and the seal member 646a. The seal member 646a is an example of a seal member. The retainer 146 is an example of a retaining member.

### (Retainer)

The retainer 146 is, for example, made of a metal such as stainless steel or the like and has conductivity. The retainer 146 includes the pressing surface 146d that abuts on a shaft-side surface 246d (a Y2-side surface) of the seal member 646a and presses the shaft-side surface 246d of the seal member 646a in the D direction in an area other than a center portion of the shaft-side surface 246d.

### (Seal Member)

The seal member 646a is provided in the proximal clevis 45 and is configured to prevent the gas filled in the body cavity of the patient P from leaking out of the body from the treatment portion via the shaft 42.

Specifically, the seal member 646a is formed of an elastically deformable conductive material. The seal member 646a is made by adding carbon or the like in an elastomer such as silicone rubber or the like. The seal member 646a has a thickness in the Y direction. The seal member 646a is compressed at the Y1-side end of the inner space 45d of the proximal clevis 45. The seal member 646a is in close contact with the circumferential wall portion 45b and the partition wall portion 452 of the connection base 45a of the proximal clevis 45. Note that the other configurations in a second embodiment are the same as those in a first embodiment described above.

### (Electrical Connection)

Here, an electrical connection from the electric wire 47 to the end effector 43 in the surgical instrument 604 is described.

The seal member 646a has conductivity. The electric wire 47 is connected to the retainer 146 at the connector portion 247 of the electric wire 47, and thus supplies the electrical energy to the end effector 43 through the retainer 146, the seal member 646a, the proximal clevis 45, and the distal clevis 44.

With this, unlike a first embodiment, the electric current can be flown to the proximal clevis 45 not only through the retainer 146 but also through the seal member 646a. Therefore, the electrical energy can be supplied to the end effector 43 more stably.

Specifically, the electric wire 47 and the retainer 146 are electrically connected. The end portion of the connector portion 247 the end effector 43 side is caulked in the through hole 146c. That is, the end effector-side end 147 (the Y1 side end) of the electric wire 47 is terminated at the retainer 146. Accordingly, the electricity is flown from the electric wire 47 to the retainer 146.

The retainer 146 and the seal member 646a are electrically connected. The conductive seal member 646a and the conductive retainer 146 are in closed contact with and thud connected with each other by being pressed by the shaft 42 and thus are connected to each other. With this, the electricity is flown from the retainer 146 to the seal member 646a (see the arrows in FIG. 17).

The conductive seal member 646a and the proximal clevis 45 are eclectically connected to each other in such a manner that the seal member 646a is pressed by the shaft 42 via the retainer 146 so that the seal member 646a is in close contact with the partition wall portion 452 of the connection base 45a of the proximal clevis 45. The conductive seal member 646a and the proximal clevis 45 are also eclectically connected to each other in such a manner that the seal member 646a is pressed and thus compressed by the shaft 42 via the retainer 146 so that the seal member 646a is in close contact with the circumferential wall portion 45b of the connection base 45a of the proximal clevis 45. With this, the electricity is flown from the seal member 646a to the proximal clevis 45 (see the arrows in FIG. 17).

Since the seal member 646a has conductivity, the electricity is also flown to the first and second elongate elements W1 and W2 that are in contact with the seal member 646a. With this, the electricity is flown from the first elongate element W1 to the distal clevis 44. The electricity is also flown from the second elongate elements W2 to the end effector 43.

Since the electrical connection from the proximal clevis 45 to the end effector 43 is the same as or similar to that in a first embodiment, the description thereof is omitted.

### [Third Embodiment]

With reference to FIG, 18, a configuration of a surgical instrument 704 according to a third embodiment is described. In a third embodiment, unlike a first embodiment, an electric wire 747 is connected to a seal member 746a. Note that in a third embodiment, descriptions of the same configurations as those in a first embodiment may be omitted.

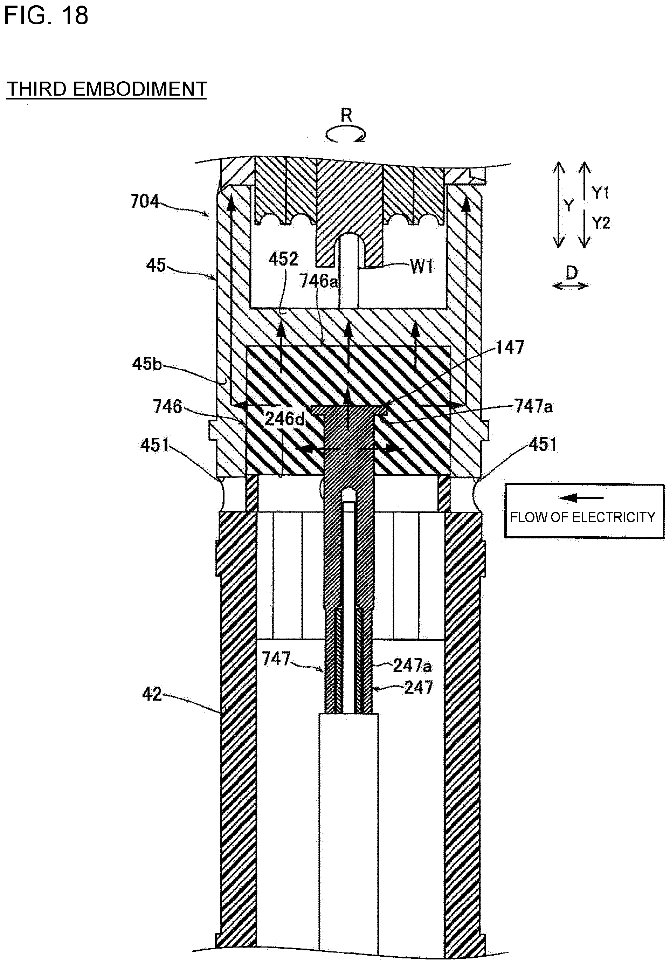

As illustrated in FIG. 18, the surgical instrument 704 according a third embodiment includes the end effector 43, the conductive distal clevis 44 supporting the end effector 43 to be rotatable about the first axis A1, and the conductive proximal clevis 45 supporting the distal clevis 44 to be rotatable about the second axis A2. The surgical instrument 704 includes the shaft 42 to be connected to the proximal clevis 45, and a sealing mechanism 746 (a sealing device 746) provided in contact with the proximal clevis 45 and including at least a part thereof having conductivity. The surgical instrument 704 includes an electric wire 747. The electric wire 747 includes an end effector-side end portion thereof connected to the sealing mechanism 746 to supply the electrical energy to the end effector 43 through the sealing mechanism 746, the proximal clevis 45, and the distal clevis 44. Here, unlike first and second embodiments described above, the sealing mechanism 746 does not include the retainer 146, but includes only the seal member 746a.

With this configuration, the sealing performance of the seal member 746a of the surgical instrument 704 can be improved.

Here, the direction in which the surgical instrument 704 (the direction in which the shaft 42 extends) is defined as the Y direction, the distal side (the side toward the end effector 43) of the surgical instrument 704 along the Y direction is defined as the Y1 side, and the opposite side of the Y1 direction is defined as the Y2 side. The radial direction of the shaft 42 is referred to as the D direction, and the circumferential direction of the shaft 42 is referred to as the R direction.

### (Sealing mechanism)

As described above , the sealing mechanism 746 does not include the retainer 146 but includes only the seal member 746a. The seal member 746a is an example of a seal member.

### (Seal Member)

The seal member 746a is provided in the proximal clevis 45 and is configured to prevent the gas filled in the body cavity of the patient P from leaking out of the body from the treatment portion via the shaft 42.

The seal member 746a is configured to be an elastically deformable conductive member. The seal member 746a contains therein carbon.

With this, since the seal member 746a can be formed as a member capable of conducting the electricity easily, the electricity can be reliably passed from the seal member 746a to the proximal clevis 45.

The seal member 746a is made by adding carbon or the like in an elastomer such as silicone rubber or the like. The seal member 746a has a thickness in the Y direction. The seal member 746a is compressed at the end portion of the inner space 45d of the proximal clevis 45 on the Y1 side. The seal member 746a is in close contact with the circumferential wall portion 45b and the partition wall portion 452 of the connection base 45a of the proximal clevis 45.

### (Electric Wire)

As illustrated in FIG. 18, the end effector-side end 147 of the electric wire 747 is formed with the connector portion 247, which is to be connected to the seal member 746a.

The tip portion (the end effector-side end portion) of the connector portion 247 is embedded in and thus connected to the seal member 746a. That is, the end effector-side end 147 (the Y1-side end) of the electric wire 747 is terminated at the seal member 746a.

With this configuration, since the connector portion 247 of the electric wire 747 and the seal member 746a can be brought into close contact with each other, the electricity can reliably flow from the electric wire 747 to the seal member 746a.

The connector portion 247 includes a retaining portion 747a that prevents the connector portion 247 from coming off from the seal member 746a toward the shaft 42 side (the Y2 side) in the axial direction of the shaft 42 (in the Y direction). The connector portion 247 is integrally formed with the seal member 746a with the retaining portion 747a of the connector portion 247 preventing the connector portion 247 from coming off from the sealing member 746a.

As a result, the connector portion 247 can be effectively prevented from coming off from the seal member 746a by means of the retaining portion 747a, so that the connection between the connector portion 247 and the seal member 746a can be more reliably maintained. Further, unlike a case where the connector portion 247 is connected to the sealing member 746a after the seal member 746a is formed, the seal member 746a and the connector portion 247 can be connected at the time of resin molding of the seal member 746a, so that the connection process to connecting the seal member 746a and the connector portion 247 can be simplified.

Specifically, the electric wire 747 and the seal member 746a are integrally formed by insert-molding the connector portion 247 into the seal member 746a. Thus, the seal member 746a and the connector portion 247 are in close contact with each other. The connector portion 247 has a substantially T shape in a cross section taken along the Y direction. Specifically, the connector portion 247 includes a connector main body portion 247a extending in the axial direction of the shaft 42 and the retaining portion 747a extending from a tip (a Y1-side end) of the connector main body portion 247a in the direction crossing the axial direction of the shaft 42. The retaining portion 747a protrudes from the tip of the connector main body portion 247a in the direction (the D direction, the X direction) orthogonal to the Y direction. Note that the other configurations in a third embodiment are the same as those in a first embodiment described above.

### (Electrical Connection)

Here, an electrical connection from the electric wire 747 to the end effector 43 in the surgical instrument 704 is described.

The sealing mechanism 746 does not include the retainer 146 pressed by the shaft 42, but includes the conductive seal member 746a disposed between the proximal clevis 45 and the shaft 42. The electric wire 747 is connected to the seal member 746a at the connector portion 247 of the electric wire 747 to supply the electrical energy to the end effector 43 through the seal member 746a, the proximal clevis 45, and the distal clevis 44.

According to this configuration, by eliminating the retainer 146, it is possible to supply the electrical energy to the end effector 43 with improved sealing performance of the seal member 746a while reducing the number of parts of the surgical instrument 704.

Specifically, by integrally forming the connector portion 247 and the seal member 746a, the electric wire 747 and the seal member 746a are in close contact with each other and thus are electrically connected to each other. With this, the electricity is flown from the electric wire 747 to the seal member 746a (see the arrows in FIG. 18).

The seal member 746a is pressed by the shaft 42 and is thus in close contact with the partition wall portion 452 of the connection base 45a of the proximal clevis 45. Thus, the seal member 746a and the proximal clevis 45 are electrically connected. Further, by being pressed by the shaft 42, the seal member 746a is compressed and brought into close contact with the circumferential wall portion 45b of the connection base 45a of the proximal clevis 45. Thus, the seal member 746a and the proximal clevis 45 are electrically connected. With this, the electricity is flown from the seal member 746a to the proximal clevis 45 (see the arrows in FIG. 18).

Since the seal member 746a has conductivity, the electricity is also flown to the first and second elongate elements W1 and W2 that are in contact with the seal member 746a. With this, the electricity is flown from the first elongate element W1 to the distal clevis 44 and is also flown from the second elongate elements W2 to the end effector 43.

Since the electrical connection from the proximal clevis 45 to the end effector 43 is the same as or similar to that in a first embodiment, the description thereof is omitted.

### [Fourth Embodiment]

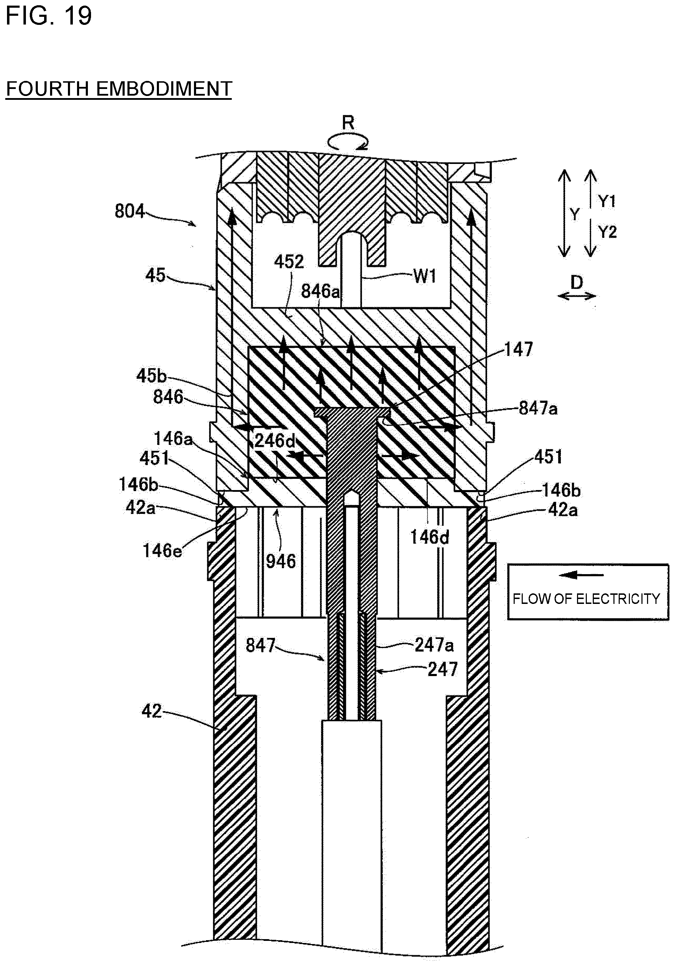

With reference to FIG. 19, a configuration of a surgical instrument 804 according to a fourth embodiment is described. In a fourth embodiment, unlike a first embodiment, a seal member 846a has conductivity and a retainer 946 does not have conductivity. In a fourth embodiment, descriptions of the same configurations as those in a first embodiment may be omitted.

As illustrated in FIG. 19, the surgical instrument 804 according a fourth embodiment includes the end effector 43, the conductive distal clevis 44 supporting the end effector 43 to be rotatable about the first axis A1, and the conductive proximal clevis 45 supporting the distal clevis 44 to be rotatable about the second axis A2. The surgical instrument 804 includes the cylindrical tubular shaft 42 to be connected to the proximal clevis 45, and a sealing mechanism 846 (a sealing device 846) provided in contact with the proximal clevis 45 and including at least a part thereof having conductivity. The surgical instrument 804 includes an electric wire 847. The electric wire 847 has an end effector-side end portion thereof connected to the sealing mechanism 846 to supply the electrical energy to the end effector 43 through the sealing mechanism 846, the proximal clevis 45, and the distal clevis 44.

With this configuration, like a first embodiment described above, the sealing performance of the sealing mechanism 846 of the surgical instrument 804 can be improved.

Here, the direction in which the surgical instrument 804 extends (the direction in which the shaft 42 extends) is defined as the Y direction, the distal side of the surgical instrument 804 (the side toward the end effector 43) along the Y direction is defined as the Y1 side, and the opposite side of the Y1 side is defined as the Y2 side. The radial direction of the shaft 42 is referred to as the D direction, and the circumferential direction of the shaft 42 is referred to as the R direction.

### (Sealing mechanism)

The sealing mechanism 846 includes the seal member 846a and the retainer 946. The seal member 846a is an example of a seal member. The retainer 946 is an example of a retaining member.

### (Retainer)

The retainer 946 is made of resin material and has thus no conductivity. The retainer 946 includes the pressing surface 146d that abuts on the shaft 42 side surface 246d (the Y2-side surface) of the seal member 846a and presses the surface 246d in the D direction in an area other than the center portion of the surface 246d of the seal member 846a.

In this way, the sealing mechanism 846 includes the insulating retainer 946 pressed by the shaft 42 and thus pressing the shaft-side surface 246d of the seal member 846a.

With this configuration, since the electric wire 847 and the proximal clevis 45 can be electrically connected via the seal member 846a which is in close contact with the proximal clevis 45, the electricity can be stably passed from the electric wire 847 to the proximal clevis 45.

### (Seal Member)

The seal member 846a is provided in the proximal clevis 45 and prevents the gas filled in the body cavity of the patient P from leaking out of the body from the treatment portion via the shaft 42.

The seal member 846a is configured to be an elastically deformable conductive member. The seal member 846a is made by adding carbon or the like in an elastomer such as silicone rubber or the like. The seal member 846a has a thickness in the Y direction. The seal member 846a is compressed at the end of the inner space 45d of the proximal clevis 45 on the Y1 side. The seal member 846a is in close contact with the circumferential wall portion 45b and the partition wall portion 452 of the connection base 45a of the proximal clevis 45.

### (Electric Wire)

As illustrated in FIG. 19, the end effector-side end 147 of the electric wire 847 is formed with the connector portion 247, which is connected to the sealing mechanism 46.

The tip portion (the end effector-side end portion) of the connector portion 247 is embedded in and thus connected to the seal member 846a. That is, the end effector-side end portion (the Y1-side end portion) of the electric wire 847 is terminated at the seal member 846a.

The connector portion 247 includes a retaining portion 847a that prevents the connector portion 247 from coming off from the seal member 846a toward the shaft 42 side (the Y2 side) in the axial direction of the shaft 42 (in the Y direction). The connector portion 247 is integrally formed with the seal member 846a with the retaining portion 847a of the connector portion 247 preventing the connector portion 247 from coming off from the sealing member 846a.

Specifically, the electric wire 847 and the seal member 846a are integrally formed by insert-molding the connector portion 247 into the seal member 846a. Thus, the seal member 846a and the connector portion 247 are in close contact with each other. The connector portion 247 has a substantially T shape in the cross section taken along the Y direction. Specifically, the connector portion 247 includes the connector main body portion 247a extending in the axial direction of the shaft 42 and the retaining portion 847a extending from the tip portion (the Y1-side end portion) of the connector main body portion 247a in the direction crossing the axial direction of the shaft 42. The retaining portion 847a protrudes from the tip portion of the connector main body portion 247a in the direction (the D direction, the X direction) orthogonal to the Y direction. Note that the other configurations in a fourth embodiment are same as those in a first embodiment described above.

### (Electrical Connection)

Here, an electrical connection from the electric wire 847 and the end effector 43 in the surgical instrument 804 is described.

The seal member 846a has conductivity. The electric wire 47 is connected to the seal member 846a at the connector portion 247 of the electric wire 47, and thus supplies the electrical energy to the end effector 43 through the seal member 846a, the proximal clevis 45, and the distal clevis 44. Here, since the retainer 946 has insulating properties, the electricity does not flow from the electric wire 847 to the retainer 946.

The conductive seal member 846a and the proximal clevis 45 are eclectically connected to each other in such a manner that the seal member 846a is pressed by the shaft 42 via the retainer 946 so that the seal member 846a is in close contact with the partition wall portion 452 of the connection base 45a of the proximal clevis 45. The conductive seal member 846a and the proximal clevis 45 are also eclectically connected to each other in such a manner that the seal member 846a is pressed and thus compressed by the shaft 42 via the retainer 946 so that the seal member 846a is in close contact with the circumferential wall portion 45b of the connection base 45a of the proximal clevis 45. With this, the electricity is flown from the seal member 846a to the proximal clevis 45 (see the arrows in FIG. 19).

Since the seal member 846a has conductivity, the electricity is also flown to the first and second elongate elements W1 and W2 that are in contact with the seal member 846a. With this, the electricity is flown from the first elongate element W1 to the distal clevis 44 and is also flown from the second elongate elements W2 to the end effector 43.

Since the electrical connection from the proximal clevis 45 to the end effector 43 is the same as or similar to that in a first embodiment, the description thereof is omitted.

### [Modifications]

It should be understood that first to fourth embodiments described above are illustrated by way of example in every respect and do not limit the invention. The scope of the invention is indicated by claims, not by explanation of the first to fourth embodiments described above, and includes equivalents to the claims and all alterations (modification) within the same.

For example, in first to fourth embodiments described above, the case has been described in which the electric wire 47 (747, 847) includes the connector portion 247. However, the invention is not limited thereto. In the invention, the electric wire may not include such a connector portion.

Also, in first and second embodiments described above, the case has been described in which the retainer 146 (retaining member) includes the through hole in which the tip portion of the connector portion 247 is caulked. However, the invention is not limited thereto. In the invention, the retaining member may be connected by means of a method other than the caulking.

Also, in third and fourth embodiments described above, the case has been described in which the connector portion 247 is electrically connected to the seal member 746a (846a) by embedding the tip portion of the connector portion 247 in the seal member 746a (846a). However, the invention is not limited thereto. In the invention, the connector portion may be in contact with the seal member and thus be electrically connected to the seal member.

Also, in third and fourth embodiments described above, the case has been described in which the connector portion 247 includes the retaining portion 747a (847a) for preventing the connector portion 247 from coming off from the seal member 746a (846a) toward the shaft 42 side in the Y direction (the axial direction of the shaft 42). However, the invention is not limited thereto. In the invention, the connector portion may not include such a retaining portion.

Also, in second to fourth embodiments described above, the case has been described in which the seal member 646a (746a, 846a) contains therein carbon. However, the invention is not limited thereto. In the invention, the conductive seal member may be produced by adding a metal fiber or the like in the seal member.

Further, in first to fourth embodiments described above, the case has been described in which the surgical instrument 4 includes the insulating cover 48 that covers from the outside in the D direction (the radial direction of the shaft 42). However, the invention is not limited thereto. In the invention, the surgical instrument may be insulated by a method other than such an insulating cover.

The disclosure includes the following itemized list:
1. A surgical instrument to be attached to a robot arm, comprising:
   an end effector;
   a conductive distal support body that supports the end effector to be rotatable about a first axis with respect to the distal support body;
   a conductive proximal support body that supports the distal support body to be rotatable about a second axis with respect to the proximal support body;
   a shaft that is formed in a tubular shape and is to be connected to the proximal support body;
   a sealing mechanism that is in contact with the proximal support body and at least a part of which has conductivity; and
   an electric wire that includes an end effector-side end portion thereof being connected to the sealing mechanism to supply electrical energy to the end effector through the sealing mechanism, the proximal support body, and the distal support body.
2. The surgical instrument according to item 1, wherein
   the sealing mechanism is provided between the shaft and the proximal support body.
3. The surgical instrument according to item 2, wherein
   the sealing mechanism comprises:
   a seal member that seals an inside of the shaft; and
   a retaining member that is pressed by the shaft to press a shaft-side surface of the seal member.
4. The surgical instrument according to item 3, wherein
   the retaining member has conductivity, and
   the end effector-side end portion of the electric wire is connected to the retaining member of the sealing mechanism, such that the electric wire supplies the electrical energy to the end effector through the retaining member, the proximal support body, and the distal support body.
5. The surgical instrument according to item 4, wherein
   the seal member is formed of an insulating material.
6. The surgical instrument according to item 4, wherein
   the seal member has conductivity, and
   the electric wire is connected to the retaining member of the sealing mechanism to supply the electrical energy to the end effector through the retaining member, the seal member, the proximal support body, and the distal support body.
7. The surgical instrument according to any one of items 3 to 6, wherein
   the proximal support body includes a cutout recessed from a shaft-side end portion of the proximal support body toward the end effector side in an axial direction of the shaft,
   the retaining member includes a pressed portion arranged in the cutout and configured to be pressed by the shaft, and
   in a state where the pressed portion of the retaining member is pressed by the shaft, the pressed portion is in contact with the cutout.
8. The surgical instrument according to item 7, wherein
   an end effector-side surface of the pressed portion of the retaining member is in surface contact with an end effector-side inner surface of the cutout of the proximal support body.
9. The surgical instrument according to any one of items 4 to 8, wherein
   the end effector-side end portion of the electric wire is formed with a connector portion to be connected to the sealing mechanism, and
   the retaining member includes a through hole in which a tip portion of the connector portion is caulked.
10. The surgical instrument according to item 9, wherein
   a shaft-side surface of the seal member is formed with an accommodation recess that is recessed toward the end effector side and accommodates therein the tip portion of the connector portion so as not to interfere with the tip portion of the connector portion.
11. The surgical instrument according to item 1 or 2, wherein
   the sealing mechanism includes a conductive seal member that seals an inside of the shaft, and
   the electric wire is connected to the seal member, such that the electric wire supplies the electrical energy to the end effector through the seal member, the proximal support body, and the distal support body.
12. The surgical instrument according to item 11, wherein
   the seal member is elastically deformable.
13. The surgical instrument according to item 11 or 12, wherein
   the seal member contains therein carbon.
14. The surgical instrument according to any one of items 11 to 13, wherein
   the sealing mechanism further includes an insulating retaining member that is pressed by the shaft to press a shaft-side surface of the seal member.
15. The surgical instrument according to any one of items 11 to 14, wherein
   the end effector-side end portion of the electric wire is formed with a connector portion to be connected to the sealing mechanism, and
   a tip portion of the connector portion of the electric wire is embedded in and thus connected to the seal member.
16. The surgical instrument according to item 15, wherein
   the connector portion includes a connector main body portion extending in an axial direction of the shaft and a retaining portion extending from the connector main body portion in a direction crossing the axial direction of the shaft.
17. The surgical instrument according to item 15 or 16, wherein
   the seal member is integrally formed with the connector portion of the electric wire.
18. The surgical instrument according to any one of items 1 to 17, further comprising:
   an elongate element for rotating the distal support body about a shaft portion with respect to the proximal support body, wherein
   the proximal support body includes a shaft hole that supports the shaft portion, and
   in a state where the distal support body is pulled toward the shaft by the elongated element, an inner surface of the shaft hole and the shaft portion are in contact with each other, to electrically connect the proximal support body to the end effector via the distal support body.
19. The surgical instrument according to any one of items 1 to 18, further comprising:
   an insulating cover that covers the distal support body, the proximal support body, and the sealing mechanism from an outside in a radial direction of the shaft.
20. The surgical instrument according to any one of items 1 to 19, wherein
   the surgical instrument comprises a monopolar electrosurgical instrument comprising the electric wire as a single electric wire.

## Claims

1. A surgical instrument to be attached to a robot arm, comprising:
an end effector;
a conductive distal support body that supports the end effector to be rotatable about a first axis with respect to the distal support body;
a conductive proximal support body that supports the distal support body to be rotatable about a second axis with respect to the proximal support body;
a shaft that is formed in a tubular shape and is to be connected to the proximal support body;
a sealing mechanism that is in contact with the proximal support body and at least a part of which has conductivity; and
an electric wire that includes an end effector-side end portion thereof being connected to the sealing mechanism to supply electrical energy to the end effector through the sealing mechanism, the proximal support body, and the distal support body.

2. The surgical instrument according to claim 1, wherein
the sealing mechanism is provided between the shaft and the proximal support body.

3. The surgical instrument according to claim 2, wherein
the sealing mechanism comprises:
a seal member that seals an inside of the shaft; and
a retaining member that is pressed by the shaft to press a shaft-side surface of the seal member.

4. The surgical instrument according to claim 3, wherein
the retaining member has conductivity, and
the end effector-side end portion of the electric wire is connected to the retaining member of the sealing mechanism, such that the electric wire supplies the electrical energy to the end effector through the retaining member, the proximal support body, and the distal support body.

5. The surgical instrument according to claim 4, wherein
the seal member is formed of an insulating material.

6. The surgical instrument according to claim 4, wherein
the seal member has conductivity, and
the electric wire is connected to the retaining member of the sealing mechanism to supply the electrical energy to the end effector through the retaining member, the seal member, the proximal support body, and the distal support body.

7. The surgical instrument according to any one of claims 3 to 6, wherein
the proximal support body includes a cutout recessed from a shaft-side end portion of the proximal support body toward the end effector side in an axial direction of the shaft,
the retaining member includes a pressed portion arranged in the cutout and configured to be pressed by the shaft, and
in a state where the pressed portion of the retaining member is pressed by the shaft, the pressed portion is in contact with the cutout.

8. The surgical instrument according to claim 7, wherein
an end effector-side surface of the pressed portion of the retaining member is in surface contact with an end effector-side inner surface of the cutout of the proximal support body.

9. The surgical instrument according to any one of claims 4 to 8, wherein
the end effector-side end portion of the electric wire is formed with a connector portion to be connected to the sealing mechanism, and
the retaining member includes a through hole in which a tip portion of the connector portion is caulked.

10. The surgical instrument according to claim 9, wherein
a shaft-side surface of the seal member is formed with an accommodation recess that is recessed toward the end effector side and accommodates therein the tip portion of the connector portion so as not to interfere with the tip portion of the connector portion.

11. The surgical instrument according to claim 1 or 2, wherein
the sealing mechanism includes a conductive seal member that seals an inside of the shaft, and
the electric wire is connected to the seal member, such that the electric wire supplies the electrical energy to the end effector through the seal member, the proximal support body, and the distal support body.

12. The surgical instrument according to claim 11, wherein
the seal member is elastically deformable.

13. The surgical instrument according to claim 11 or 12, wherein
the seal member contains therein carbon.

14. The surgical instrument according to any one of claims 11 to 13, wherein
the sealing mechanism further includes an insulating retaining member that is pressed by the shaft to press a shaft-side surface of the seal member.

15. The surgical instrument according to any one of claims 11 to 14, wherein
the end effector-side end portion of the electric wire is formed with a connector portion to be connected to the sealing mechanism, and
a tip portion of the connector portion of the electric wire is embedded in and thus connected to the seal member.
